(19) Europäisches Patentamt
European Patent Office
Office européen des brevets

(11) Veröffentlichungsnummer: **0 474 110 B1**

(12) **EUROPÄISCHE PATENTSCHRIFT**

(45) Veröffentlichungstag der Patentschrift: **01.02.95**

(51) Int. Cl.[6]: **C07D 333/16**, C07D 207/333, C07D 409/14, C07D 405/14, C08G 61/04

(21) Anmeldenummer: **91114447.5**

(22) Anmeldetag: **28.08.91**

# (54) Arylmethylole, deren Herstellung und Verwendung.

(30) Priorität: **29.08.90 DE 4027253**

(43) Veröffentlichungstag der Anmeldung:
**11.03.92 Patentblatt 92/11**

(45) Bekanntmachung des Hinweises auf die Patenterteilung:
**01.02.95 Patentblatt 95/05**

(84) Benannte Vertragsstaaten:
**AT BE CH DE ES FR GB IT LI NL**

(56) Entgegenhaltungen:
**EP-A- 0 099 984**
**EP-A- 0 218 093**
**WO-A-87/00678**

**SYNTHETIC METALS Bd. 41, Nr. 1-2, 1991, LAUSANNE Seiten 487 - 491; H. BRÄUNLINGET AL.: 'Polydiheteroarylenemethines - a new class of conducting polymers'**

**SYNTHETIC METALS Bd. 42, Nr. 1-2, 1991, LAUSANNE Seiten 1539 - 1547; H.BRÄUNLING ET AL.: 'Polyarylmethines; synthesis and physical properties'**

(73) Patentinhaber: **WACKER-CHEMIE GMBH**
**Hanns-Seidel-Platz 4**
**D-81737 München (DE)**

(72) Erfinder: **Bräunling, Hermann, Dr.**
**Marktler Strasse 82**
**W-8263 Burghausen (DE)**
Erfinder: **Becker, Richard, Dr.**
**Ueberreiterstrasse 17**
**W-8261 Emmerting (DE)**
Erfinder: **Blöchl, Georg, Dr.**
**Rauschbergbergstrasse 7**
**W-8225 Traunreut (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 474 110 B1

## Beschreibung

Die vorliegende Erfindung betrifft bestimmte neue bis-[Di(hetero)arylhydroxymethyl](hetero)aromaten, Verfahren zu deren Herstellung und deren Polycondensation, insbesondere zu leitfähigen Polymeren.

W. Broser et al. (Tetrahedron, Vol. 31, Seite 1769 bis 1779 (1975)) haben bis-[Diarylhydroxymethyl]-aromaten der Formel A-CH(OH)-B-CH(OH)-A beschrieben. Die Reste A bedeuten dort Biphenylyl- oder Terphenylylreste, die Reste B Phenylen-, gegebenenfalls alkylierte Biphenylenreste, Terphenylen-, Naphthylen- und Phenylen-alkylen-phenylenreste.

Leitfähige Polypyrrole und deren Copolymere sind u.a. in EP-A-99 984 beschrieben. In EP-A-218 093 sind Polymere mit konjugierten Doppelbindungen geschildert, in denen (hetero)aromatische Kerne jeweils durch ein Kohlenstoffatom miteinander verbunden sind. Gemäß WO 87/00678 sind u.a. Polymere herstellbar, die aus gleichen Heteroaromaten bestehen, welche abwechselnd direkt oder über eine Methingruppe miteinander verknüpft sind.

Aus Rec. Trav. Chim. Pays-Bas 92, 961-969 (1973) sind die Verbindungen P,P'- und m,m'- Bis-[hydroxy(phenyl)methyl]-biphenyl als Zwischenprodukte zur Herstellung von Triphenylphosphoniumyliden bekannt.

Aufgabe der vorliegenden Erfindung war die Synthese neuer Stoffe, insbesondere neuer Zwischenprodukte zur Herstellung von elektrisch leitfähigen oder nichtlineare optische Eigenschaften besitzender Polymere. Ferner war es Aufgabe der vorliegenden Erfindung, ein neues Verfahren zur Herstellung von Polymeren bereitzustellen. Desgleichen war es Aufgabe der vorliegenden Erfindung, neue Polymere darzustellen. Schließlich war es Aufgabe der vorliegenden Erfindung, dünne Schichten auf Substraten herzustellen, die für den Einsatz der Polymere für optische, elektrooptische und elektronische Zwecke geeignet sind.

Mit Hilfe der Polymere bzw. ihrer Ausgangsstoffe ist es möglich, auch Polymerbeschichtungen mit einer Dicke von unter 1 µm herzustellen, was bisher aufgrund der Flüchtigkeit und/oder Kristallisationstendenz der Ausgangsstoffe schwierig oder unmöglich war.

Ferner können die Polymere auch mit weniger aggressiven Kondensationsmitteln als beispielsweise $POCl_3$ hergestellt werden. Dies ist von Vorteil, da solche starken Kondensationsmittel oft Anlaß zu unerwünschten Nebenreaktionen geben.

Die vorstehend genannten Aufgaben werden durch die vorliegende Erfindung gelöst durch Verbindungen der Formel (1)

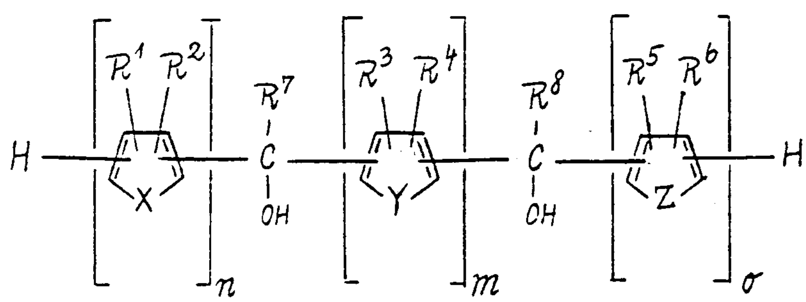 (1),

worin bedeuten

| | |
|---|---|
| **$R^1$, $R^2$, $R^3$, $R^4$, $R^5$** und **$R^6$** | gleiche oder verschiedene ringständige Reste, nämlich Wasserstoffatome, Halogenatome, geradkettige oder verzweigte $C_1$- bis $C_6$-Alkylreste, $C_1$-bis $C_6$-Alkylcarbonsäurereste oder deren Ester mit $C_1$-bis $C_4$-Alkanolen, $C_1$-bis $C_6$-Alkylaminoreste, Nitro- oder Cyanogruppen, wobei mindestens zwei der Reste **$R^1$, $R^2$, $R^3$, $R^4$, $R^5$** und **$R^6$** paarweise miteinander verknüpft sein können und eine Trimethylen- oder Tetramethylengruppe ausbilden oder mit dem Ring, an dem sie gebunden sind einen mehrkernigen (Hetero)-aromaten bilden; |
| **X, Y** und **Z** | gleiche oder verschiedene zweiwertige Reste -O-, -S-, -N($R^9$)- oder -C($R^{10}$)=C($R^{11}$)-, unter der Bedingung, daß mindestens einer der Reste X, Y und Z kein Rest der Formel -C($R^{10}$)=C($R^{11}$)- ist, und worin |
| **$R^9$** | ein Wasserstoffatom, ein verzweigter oder unverzweigter $C_1$-bis $C_8$-Alkylrest oder ein gegebenenfalls substituierter Phenylrest ist, |
| **$R^{10}$** und **$R^{11}$** | jeweils gleiche oder verschiedene Reste sind, welche eine der Bedeutun- |

| | |
|---|---|
| | gen von $R^1$ haben, |
| **$R^7$** und **$R^8$** | gleiche oder verschiedene Reste, nämlich Wasserstoffatome, $C_1$- bis $C_6$-Alkyl- oder Phenylreste, |
| **n**,**m** und **o** | gleiche oder verschieden ganze Zahlen im Wert von 1 bis 10, |

und die Umsetzung dieser Verbindungen in Gegenwart von wasserabspaltenden Mitteln zu Polymeren.

Bevorzugt als Verbindungen der Formel (1) sind solche der Formel (2)

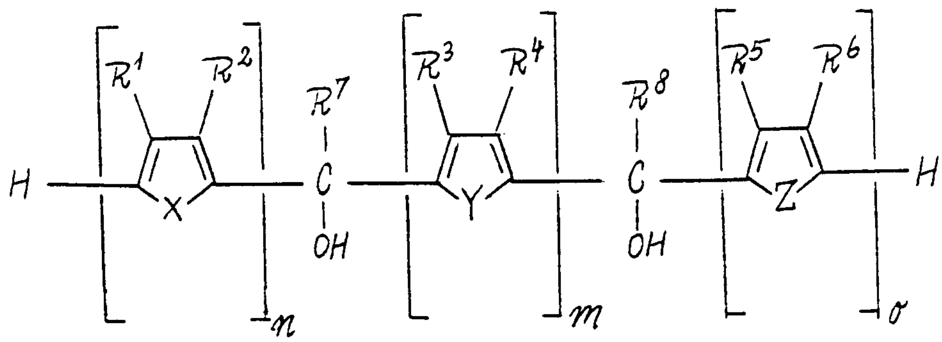

(2),

worin **$R^1$**, **$R^2$**, **$R^3$**, **$R^4$**, **$R^5$**, **$R^6$**, **$R^7$**, **$R^8$**, **X**, **Y**, **Z**, **m**, **n** und **o** die unter Formel (1) angegebenen Bedeutungen haben.

Vorzugsweise nehmen in den oben genannten und den nachstehenden Formeln **n**, **m** und **o** Werte von 1 bis 4 ein, insbesondere jeweils gleiche oder verschiedene Werte von 1 oder 2. Vorzugsweise sind die Werte für **n** und **o** gleich. Vorzugsweise sind die Reste **X**, **Y** und **Z** in den oben genannten und den nachstehenden Formeln Reste der Formel -O-, -S-, -NH- und -N($CH_3$)-. Vorzugsweise sind die Reste **X** und **Z** in den oben genannten und den nachstehenden Formeln identisch. Vorzugsweise sind in den oben genannten und den nachstehenden Formeln die Reste **$R^7$** und **$R^8$** jeweils Wasserstoffatome.

Vorzugsweise sind die Reste **$R^1$**, **$R^2$**, **$R^3$**, **$R^4$**, **$R^5$** und **$R^6$** in den obengenannten und den nachstehenden Formeln Wasserstoffatome, $C_1$- bis $C_4$-Alkylreste oder mindestens zwei der Reste **$R^1$**, **$R^2$**, **$R^3$**, **$R^4$**, **$R^5$** und **$R^6$** paarweise miteinander verknüpft, so daß sie mit dem Ring, an dem sie gebunden sind einen mehrkernigen (Hetero)aromaten bilden. Insbesondere sind die Reste **$R^1$**, **$R^2$**, **$R^3$**, **$R^4$**, **$R^5$** und **$R^6$** Wasserstoffatome oder $C_1$-bis $C_4$-Alkylreste. Vorzugsweise sind mindestens einer der Reste **$R^1$** und **$R^2$**, mindestens einer der Reste **$R^3$** und **$R^4$** und/oder mindestens einer der Reste **$R^5$** und **$R^6$** ein Wasserstoffatom. Bevorzugt als Verbindungen der Formel (1) bzw. (2) sind solche der Formel (8):

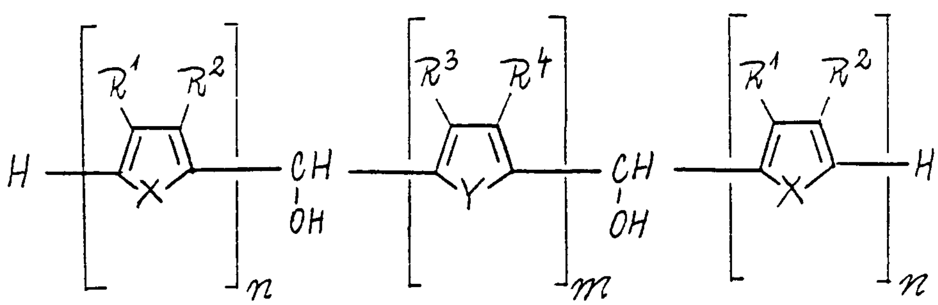

(8),

worin **$R^1$**, **$R^2$**, **$R^3$**, **$R^4$**, **X**, **Y**, **m** und **n** die unter Formel (1) angegebenen Bedeutungen haben.

Insbesondere bevorzugt als Verbindungen der Formel (1), (2) bzw. (8) sind Verbindungen der Formel (3):

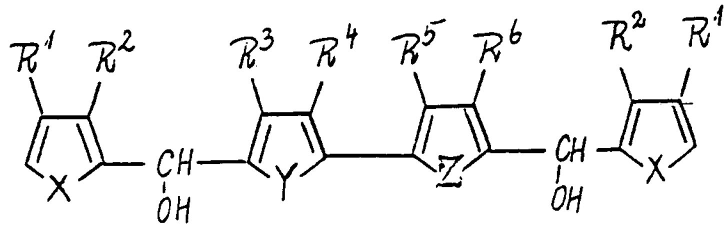

(3),

worin **X,Y, Z, $R^1$, $R^2$, $R^3$, $R^4$, $R^5$** und **$R^6$** die oben genannten Bedeutungen haben.

Die erfindungsgemäßen neuen Arylmethylole der Formel (1), (2), (3) bzw. (8) können hergestellt werden durch Umsetzung von Verbindungen der Formel (4) mit Verbindungen der Formeln (5) und (6)

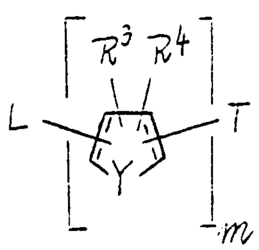

(4),

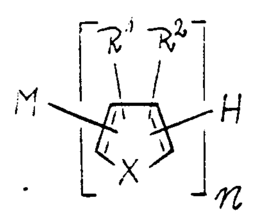

(5),

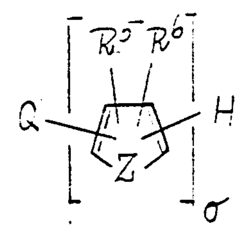

(6),

worin **entweder**

beide Reste **L** und **T** Reste aus der Gruppe der Wasserstoffatome, Metallatome und Reste der Formel -MgI, -MgBr und -MgCl sind und

**M** ein Rest der Formel $-C(R^7)=O$ und

**Q** ein Rest der Formel $-C(R^8)=O$ ist

**oder**

beide Reste **M** und **Q** Reste aus der Gruppe der Wasserstoffatome, Metallatome und Reste der Formel -MgI, -MgBr und -MgCl sind und

**L** ein Rest der Formel $-C(R^7)=O$ und

**T** ein Rest der Formel $-C(R^8)=O$ ist und

worin **$R^1$, $R^2$, $R^3$, $R^4$, $R^5$, $R^6$, $R^7$, $R^8$, X, Y, Z, m, n** und **o** die unter Formel (1) angegebenen Bedeutungen haben.

Es kann eine Verbindung der Formel (4), es kann auch ein Gemisch von mindestens zwei Verbindungen der Formel (4) im erfindungsgemäßen Verfahren eingesetzt werden. Es kann eine Verbindung der Formel (5) und /oder eine Verbindung der Formel (6), es können auch Gemische von mindestens zwei Verbindungen der Formel (5) und/oder mindestens zwei Verbindungen der Formel (6) eingesetzt werden. Auch alle anderen in diesem oder in nachfolgend geschilderten Verfahren eingesetzten Reaktionsteilnehmer, Katalysatoren, Lösungsmittel, Schutzgase, Dotierungsmittel, Beschichtungssubstrate, weitere Polymere, Weichmacher und Hilfsstoffe jeglicher Art können jeweils einzeln oder im Gemisch von mindestnes zwei Stoffen dieser Art eingesetzt werden.

Vorzugsweise werden die Reste der oben genannten Formeln (5) und (6) so gewählt, daß die Verbindung der Formel (5) mit der der Formel (6) identisch ist.

Das erfindungsgemäße Verfahren wird also vorzugsweise durchgeführt durch Umsetzung von Verbindung der Formel (9) mit Verbindung der Formel (10):

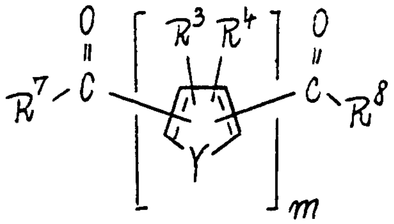

(9)

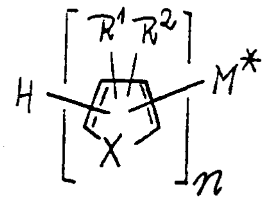

(10)

oder durch Umsetzung von Verbindung der Formel (11) mit Verbindung der Formel (12):

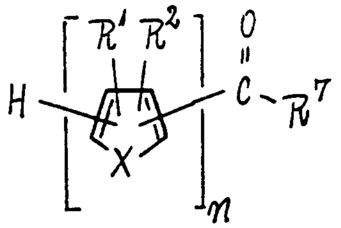

(11)

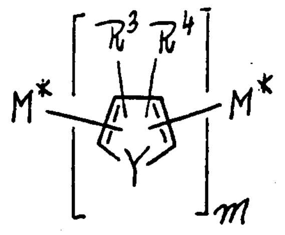

(12)

wobei in den oben aufgeführten Formeln (9), (10), (11) und (12) die Reste $\mathbf{R^1}$, $\mathbf{R^2}$, $\mathbf{R^3}$, $\mathbf{R^4}$, $\mathbf{R^7}$, $\mathbf{R^8}$, **X, Y, m** und **n** die unter Formel (1) angegebenen Bedeutungen haben und
**M*** jeweils gleiche oder verschiedene Reste bedeutet, welche ausgewählt sind aus der Gruppe der Wasserstoffatome, Metallatome, vorzugsweise Alkalimetallatome, insbesondere Lithium, und Reste der Formel -MgCl, -MgBr und -MgI.
Vorzugsweise sind die Reste **M*** jeweils gleiche Reste. Falls die Reste **M*** jeweils Wasserstoffatome sind, so wird die Reaktion vorzugsweise in Gegenwart von Säuren oder Basen durchgeführt. Vorzugsweise jedoch sind die Reste **M*** Metallatome, insbesondere Alkalimetallatome, oder Reste der Formel -MgBr, -MgCl oder -Mg-I. Besonders Bevorzugter Rest **M*** ist das Lithiumatom.

Vorzugsweise werden im erfindungsgemäßen Verfahren die Ausgangsstoffe in solchen Mengen eingesetzt, daß das Verhältnis der Summe der Molzahlen der Verbindungen der Formeln (5) und (6) zur Molzahl der Verbindungen der Formel (4) von 20 : 1 bis 0,1 : 1, insbesondere von 1,5 : 1 bis 2,5 zu 1 beträgt. In gewissen Fällen kann ein großer Überschuß einer preiswerten oder leicht zu entfernenden Komponente sinnvoll sein, damit die Reaktion im wesentlichen vollständig verläuft.

Das erfindungsgemäße Verfahren wird vorzugsweise bei Temperaturen von -80 °C bis +200 °C, besonders vorzugsweise von - 40 °C bis +100 °C, insbesondere von -20 °C bis +30 °C, durchgeführt.

Die Umsetzung kann jeweils beim Druck der umgebenden Atmosphäre erfolgen, also bei etwa 0,1 MPa (absolut). Es können auch höhere Drücke, beispielsweise bis 2 MPa (abs.) angewandt werden. Niedrigere Drücke, beispielsweise bis minimal 1 Pa (abs.) werden angewandt, insbesondere wenn Substanzen, wie Ausgangsstoffe, Nebenprodukte, Hilfsstoffe, Lösungsmittel und dergleichen während der Reaktion aus dem Reaktionsgemisch abdestilliert werden sollen.

Das erfindungsgemäße Verfahren kann in Anwesenheit oder in Abwesenheit von Lösungsmitteln durchgeführt werden. Falls Lösungsmittel verwendet werden, was bevorzugt ist, sind Lösungsmittel oder Lösungsmittelgemische mit einem Siedepunkt bzw. Siedebereich von bis zu 120° C bei 0,1 MPa bevorzugt. Beispiele für solche Lösungsmittel sind Wasser; Alkohole, wie Methanol, Ethanol, n-Propanol, iso-Propanol; Ether, wie Dioxan, Tetrahydrofuran, Diethylether, Diethylenglycoldimethylether; chlorierte Kohlenwasserstoffe, wie Dichlormethan, Trichlormethan, Tetrachlormethan, 1,2-Dichlorethan, Trichlorethylen; Kohlenwasserstoffe, wie Pentan, n-Hexan, Hexan-Isomerengemische, Heptan, Oktan, Waschbenzin, Petrolether, Benzol, Toluol, Xylole; Ketone, wie Aceton, Methylethylketon, Methylisobutylketon; Schwefelkohlenstoff und Nitrobenzol, oder Gemische dieser Lösungsmittel.

Die Bezeichnung Lösungsmittel bedeutet nicht, daß sich alle Reaktionskomponenten in diesem lösen müssen. Die Reaktion kann auch in einer Suspension oder Emulsion eines oder mehrerer Reaktionspartner

durchgeführt werden . Die Reaktion kann auch in einem Lösungsmittelgemisch mit einer Mischungslücke ausgeführt werden, wobei in jeder der Mischphasen jeweils mindestens ein Reaktionspartner löslich ist.

Falls mindestens einer der Reste **L, T, M, Q** und **M*** in den oben genannten Formeln (4), (5), (6), (10) und (12) kein Wasserstoffatom bedeutet, was bevorzugt ist, so wird das Verfahren in Abwesenheit von protischen Lösungsmitteln durchgeführt. Bevorzugte Lösungsmittel sind in diesem Fall insbesondere Diethylether, Tetrahydrofuran, 1,4-Dioxan, Benzol und Toluol.

Es kann sinnvoll sein, die Reaktion bei Abwesenheit von wesentlichen Mengen an Wasser und/oder in Anwesenheit von Schutzgas durchzuführen. Dies ist besonders ratsam, falls mindestens einer der Reaktanden ein Metallatom, einen Rest der Formel -MgBr oder -MgCl aufweist. In diesem Falle sollte das Schutzgas auch im wesentlichen frei von $CO_2$ sein. Beispiele für solche Schutzgase sind Stickstoff, Argon und deren Gemische.

Die Reaktanden der Formeln (4), (5), (6), (9), (10), (11) und (12) sind teilweise käuflich, teilweise lassen sie sich nach bekannten Verfahren herstellen. So sind die Heteroarylaldehyde der Formel (11) mit n = 1 zum großen Teil käuflich. Biheteroaryldialdehyde der Formel (9) (m = 2) lassen sich beispielsweise durch Umsetzung der entsprechenden Halogenheteroarylaldehyden mit Kupferpulver (Ullmann-Reaktion) herstellen. Die Herstellung der Lithium- bzw. Grignardverbindungen der Formeln (10) und (12) gelingt durch Umsetzung der entsprechenden Halogenverbindungen der Heteroaromaten und Biheteroaromaten auf an sich bekannte Weise mit n-Butyllithium bzw. Magnesiummetall bzw. durch Umsetzung der Heteroaromaten und Biheteroaromaten mit n-Butyllithium.

<u>Herstellung von Polymeren</u>

Aus den oben genannten Verbindungen der Formeln (1), (2), (8) und (3) lassen sich durch Polykondensation Polymere mit (Hetero-)arylenmethineinheiten herstellen. Die Polykondensation wird in Gegenwart eines Kondensationsmittels aus der Gruppe der Säurechloride, Säureanhydride und starken Säuren, Basen und hygroskopischen Verbindungen durchgeführt. Beispiele für hygroskopische Verbindungen sind $CaCl_2$, KOH, $CuSO_4$, $Co(NO_3)_2$ und ähnliche mehr jeweils in getrockneter Form ohne Kristallwasser. Beispiele für solche Säurechloride sind $SOCl_2$, $POCl_3$, $PCl_5$ und p-Toluolsulfonsäurechlorid; Beispiele für solche Säureanhydride sind Acetanhydrid, Trifluormethansulfonsäureanhydrid, $P_4O_{10}$,$SO_3$ und Trifluoressigsäureanhydrid. Beispiele für solche starken Säuren sind p-Toluolsulfonsäure, Schwefelsäure, $C_1$-bis $C_8$-Alkyl- oder Arylsulfonsäuren, Fluorsulfonsäure und Chlorsulfonsäure. Beispiele für Basen sind Lewis-Basen, wie das Hydroxyl-Ion, das Methanolat- und das Ethanolat-Ion und das Isopropanolat-Ion, Halogenid-Ionen, wie Fluorid-, Chlorid-, Bromid- und Jodid-Ionen, das Cyanid-Ion, das Hydrid-Ion, Kohlenmonoxid, Ammoniak, Amine, Ether, wie Diethylether, Tetrahydrofuran und 1,3-Dioxan, und Wasser; Brønstedt-Basen, wie Alkali- und Erdalkalihydroxide, wie LiOH, NaOH, KOH, RbOH, CsOH, $Mg(OH)_2$, $Ca(OH)_2$, $Sr(OH)_2$, $Ba(OH)_2$, und Amide, wie Natrium- und Kaliumamid, Hydride, wie Natrium-, Kalium- und Kalziumhydrid.

Falls das Kondensationsmittel im Reaktionsgemisch nicht oder nur wenig löslich ist, kann es vorteilhaft sein, einen geeigneten lösungsvermittelnden, den Phasentransfer beschleunigenden und/oder das Kondensationsmittel dispergierenden Stoff zuzusetzen oder das Kondensationsmittel in entsprechender Formulierung einzusetzen.

Das Kondensationsmittel kann zusammen mit abgespaltenem Kondensat nach der Kondensation entfernt werden, insbesondere, sofern möglich, destillativ.

Falls im erfindungsgemäßen Verfahren Kondensationsmittel verwendet werden, so sind solche bevorzugt, die bei einer Temperatur von 80 ° C und einem Druck von 0,1 MPa gasförmig sind. Besonders bevorzugt ist es, Kondensationsmittel in Mengen von 0,5 Mol bis 2 Mol, insbesondere von 1 Mol bis 2 Mol pro Mol der eingesetzten Verbindung(en) der Formel (1) einzusetzen. Falls die Kondensation durch Kondensationsmittel beschleunigt werden soll, so erreicht man auch mit katalytischen Mengen an Kondensationsmitteln eine hohe Reaktionsgeschwindigkeit, wenn man das im Laufe der Reaktion entstehende Wasser durch geeignete physikalische Verfahren, beispielsweise durch azeotrope Destillation, aus dem Reaktionsgemisch entfernt.

Die erfindungsgemäße Polykondensation kann in Gegenwart von Lösungsmittel durchgeführt werden. Für die Auswahl und Beispiele solcher Lösungsmittel gilt entsprechend, was oben zu im Verfahren zur Herstellung der Arylmethylole verwendbaren Lösungsmitteln erwähnt wurde. Die Verwendung protischer Lösungsmittel ist jedoch in vielen Fällen nicht nachteilig.

Bevorzugte Lösungsmittel sind Aceton, Methanol, Ethanol, THF, Methylenchlorid und Chloroform.

Als Produkt der erfindungsgemäßen Polykondensation erhält man schwarze, pulverförmige Polymere, welche aus Einheiten der Formel (13) aufgebaut sind:

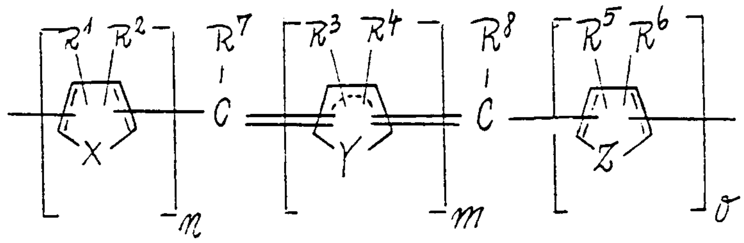

(13),

worin **R¹**, **R²**, **R³**, **R⁴**, **R⁵**, **R⁶**, **R⁷**, **R⁸**, **X, Y, Z, m, n** und **o** die unter Formel (1) angegebenen Bedeutungen haben und das gestrichelte Kreissegment im mittleren Arylring der Formel für eine konjugierte Doppelbindung steht.

Erfindungsgemäß hergestellte Polymere, in denen mindestens einer der Reste $\mathbf{R^1}$, $\mathbf{R^2}$, $\mathbf{R^3}$, $\mathbf{R^4}$, $\mathbf{R^5}$ und $\mathbf{R^6}$ ein Alkylrest ist, sind oft in organischen Lösungsmitteln, wie $CH_2Cl_2$ und Tetrahydrofuran löslich. Die übrigen der erfindungsgemäßen Polymere sind im allgemeinen in den gängigen organischen Lösungsmitteln nicht merklich löslich.

Durch die erfindungsgemäße Polykondensation von Verbindungen der Formel (1) zu aus Einheiten der Formel (13) aufgebauten Polymeren können dünne Polymerschichten hergestellt werden, u.a. Schichten,die dünner sind als ein Mikrometer. Die Art und Stärke des Kondensationsmittels, sofern überhaupt ein solches benötigt wird, ist abhängig von der Struktur und Reaktivität der eingesetzten Verbindungen der Formel (1). Im allgemeinen benötigen die Verbindungen der Formel (1), wenn überhaupt, weniger starke und damit weniger aggressive Kondensationsmittel und können in Schichten unter ein µm kondensiert werden. Beispiel für ein solches weniger aggressives Kondensationsmittel, das in der erfindungsgemäßen Polykondensation mit Erfolg eingesetzt wurde, ist Trifluoressigsäuredampf. Schichten können u.a. hergestellt werden indem man eine bestimmte Menge eines Kondensationsmittels zusammen mit einem oder mehreren Verbindungen der Formel (1) ggf. unter Kühlung löst, die Lösung auf ein Substrat aufträgt und das Lösungsmittel abdestilliert oder verdampfen läßt.

Die so erhältlichen Polymere sind elektrisch leitende bzw. halbleitende Verbindungen.

Durch Zusatz von bekannten Dotierstoffen kann die Leitfähigkeit der Polymeren noch erhöht werden.

Dotierungsmittel sind Alkalimetalle, wie Natrium oder Kalium; Protonensäuren wie $H_2SO_4$, $HClO_4$, $H_2Cr_2O_7$, HJ und $HNO_3$; Lewis-Säuren wie $SbCl_5$, $AsCl_5$, $TiCl_4$, $FeCl_3$, $SnCl_4$, $ZnCl_2$, $AsF_5$ und Halogen, wie z.B. Jod. Die Behandlung der erfindungsgemäßen Zusammensetzungen mit Dotierungsmittel (n) wird im allgemeinen so durchgeführt, daß man die Dämpfe oder Lösungen des Dotierungsmittels auf die Polymeren einwirken läßt. Meist arbeitet man bei etwa 10 bis 30 °C, meist unter Feuchtigkeitsausschluß, oft unter Luftausschluß. Die dotierten Polymere enthalten vorzugsweise von 0 bis 50, besonders vorzugsweise von 0,01 bis 30, insbesondere von 0,1 bis 20 Gew.-% Dotierungsmittel.

Dotierungsmittel können vor, während oder nach der Polykondensation zugegeben werden. Wird Kondensationsmittel zur erfindungsgemäßen Polykondensation eingesetzt, so kann Dotierungsmittel vor, während oder nach der Zugabe von Kondensationsmittel zugegeben werden.

Beschichtet man Oberflächen von Silicium, Glas, Quarz mit Lösungen oder Schmelzen der Verbindungen der Formel (1) nach an sich bekannten Verfahren und setzt die beschichteten Träger ggf. nach Verdampfen des Lösungsmittels gas- oder dampfförmigen Kondensationsmitteln aus, so bilden sich zunächst starke meist blau gefärbte Überzüge, die sich nach einiger Zeit dunkelbraun bis schwarz verfärben. Manche dieser Überzüge weisen einen metallischen Oberflächenglanz auf. Die Beschichtungen mit reinen Verbindungen der Formel (1) haben in den meisten Fällen den Nachteil, daß die Filme schlecht haften und während des Kondensationsvorganges schrumpfen und einreissen. Löst man jedoch die Verbindungen der Formel (1) zusammen mit einem weiteren Polymer - d.h. einem Polymer, das nicht durch Polykondensation von Verbindungen der Formel (1) herstellbar ist, vorzugsweise einem oder mehreren thermoplastischen Polymeren - dann erhält man gut haftende und stabile Filme. Als Polymere können prinzipiell alle löslichen oder durch Lösungsmittel oder die Reaktanden quellbaren Polymere eingesetzt werden.

Auf diese Weise kann man Zusammensetzungen erhalten, indem das nach dem erfindungsgemäßen Verfahren herstellbare Polymer feinverteilt in dem weiteren Polymer bzw. in den weiteren Polymeren vorliegt.

Beispiele für solche geeigneten weiteren Polymere sind organische synthetische Polymere, insbesondere thermoplastische Polymere, wie Polyvinylchlorid, Polyethylen, Polypropylen, Polyvinylacetat, Polycarbonat, Polyacrylat, Polymethacrylat, Polymethylmethacrylat, Polystyrol, Polyacrylnitril, Polyvinylidenchlorid,

Polyvinylfluorid, Polyvinylidenfluorid, Polyvinylidencyanid, Polybutadien, Polyisopren, Polyether, Polyester, Polyamid, Polyimid, Silikone, Polyvinylalkohol, Polyvinylpyrrolidon, Polyacrylamid, Polyethylenglykol und deren Derivate und ähnliche einschließlich Copolymere wie Styrol-Acrylat Copolymere, Vinylacetat-Acrylat Copolymere und Ethylen-Vinylacetat Copolymere sowie natürliche Polymere wie Cellulose, Stärke, Casein und natürliches Gummi, sowie halbsynthetische hochmolekulare Verbindungen wie Cellulosederivate, z. B. Methylcellulose, Hydroxymethylcellulose und Carboxymethylcellulose.

Der Anteil von weiterem, vorzugsweise thermoplastischem Polymer, bezogen auf das Gesamtgewicht von Verbindungen der Formel (1), beträgt vorzugsweise 10 Gewichtsprozent bis 2500 Gewichtsprozent, insbesondere 20 Gew.% bis 400 Gew.%.

Die Darstellung von leitfähigem Polymer in einer Matrix von weiterem, vorzugsweise thermoplastischem Polymer ist in EP-A-357 059 (entspricht U.S.-Patentanmeldung Nr.357 059 vom 23. 8. 89) beschrieben.

## Neue leitfähige Polymere

Durch Polykondensation von Verbindungen der Formel (1) sind u.a. auch Polymere herstellbar, welche Einheiten der Formel (7) enthalten:

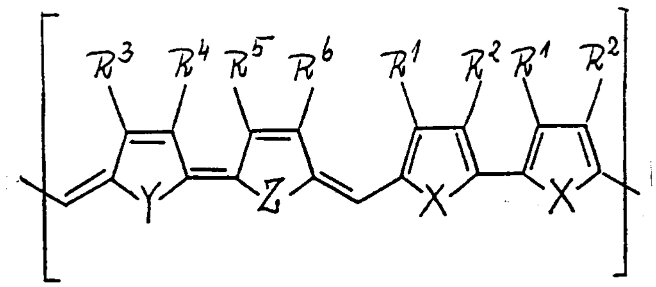

(7),

worin **$R^1$, $R^2$, $R^3$, $R^4$, $R^5$, $R^6$, X, Y, Z** die unter Formel (1) angegebenen Bedeutungen haben, mit der Maßgabe, daß mindestens einer der gemäß Formel (7) durch eine Gruppe der Formel =CH- verbundenen Ringe sich die durch mindestens einen der Reste **$R^1$, $R^2$, $R^3$, $R^4$, $R^5$, $R^6$, X, Y, Z** von den übrigen unterscheidet.

Vorzugsweise sind dies Polymere der oben genannten Formel (7), für die **X=Y=Z** nicht gilt.

Polymere, welche Einheiten der Formel (7) enthalten, sind neu. Solche Polymere können hergestellt werden durch Polykondensation von Verbindungen der Formel (3) mit der Maßgabe, daß nicht gilt **X=Y=Z**.

Vorzugsweise enthalten die neuen Polymere mindestens 10%, besonders vorzugsweise mindestens 30%, insbesondere mindestens 50%, speziell mindestens 80%, jeweils bezogen auf das Gesamtgewicht des Polymers, Einheiten der Formel (7).

Die neuen Polymere und diese Polymere enthaltende Zusammensetzungen können zusätzlich Dotierungsmittel enthalten. Die Polymere können auch feinverteilt in einem weiteren, vorzugsweise thermoplastischen Polymer vorliegen.

Was die Dotierungsmittel und weitere Polymere, Herstellungsverfahren der neuen Polymere u.a.m. betrifft, so gilt sinngemäß das im vorhergehenden Kapitel "Herstellung von Polymeren" erwähnte.

## Verwendung

Die Polarisierung gemäß Formel (1) die Reste X, Y und Z enthaltenden Ringe wirkt sich auf die optischen und elektrischen Eigenschaften aus. Will man möglichst hohe elektrische Leitfähigkeiten, so wird man Verbindungen einsetzen, für die gilt X=Y=Z. Beim Einsatz im optischen Bereich kann es jedoch wünschenswert sein, die Absorption in bestimmte Wellenlängenbereiche zu verschieben, was bei geeigneter Auswahl von X, Y und Z möglich ist. Zum Einsatz für optische Zwecke kann es auch wünschenswert sein, als mindestens einen der Reste **$R^1$, $R^2$, $R^3$, $R^4$, $R^5$, $R^6$** in Formel (1) elektronenschiebende Gruppen z. B. Amino, Alkylamino oder Dialkylamino oder elektronenziehende Gruppen z. B. Nitro oder Nitrilgruppen einzuführen.

Die erfindungsgemäßen Verbindungen eignen sich hervorragend zur Herstellung von dünnen Schichten, die durch geeignete Kondensationsmittel polykondensiert und in Schichten mit besonderen elektrischen und optischen Eigenschaften überführt werden können. Durch Einsatz der bekannten Maskentechnik können damit auch Strukturen wie Leiterbahnen oder kleine Strukturen auf Trägern, die als integrierte Sensoren,

Transistoren, Photozellen oder sonstige optische und elektrooptische Schalter dienen, erzeugt werden. Die nach dem erfindungsgemäßen Verfahren hergestellten Polymeren und die daraus erzeugten Schichten haben vielfältige optische, photoelektrische und elektrische Eigenschaften. Sie haben z. T. gute elektrische Leitfähigkeiten, sind Photoleiter und haben je nach Struktur ein Bandgap bis unter ein eV. d. h. die Lichtempfindlichkeit reicht bis ins infrarote Spektralgebiet. Fig. 1 zeigt ein UV/VIS/IR-Spektrum des Kondensationsproduktes hergestellt nach Beispiel 12.

Polymere, welche Einheiten der Formel (13) enthalten, haben nichtlineare optische Eigenschaften. Beschichtungen auf Quarz mit dem Kondensationsprodukt der gemäß Beispiel 1 herstellbaren Verbindung zeigen ein ausgeprägtes nichtlineares Verhalten (Abb. 2). Die gemessenen Werte der nichtlinearen Suszeptibilität 3. Ordnung liegen in der Größenordnung von $10^{-10}$ esu. Die beobachteten Relaxationszeiten im Pikosekundenbereich belegen die Beteiligung resonanter Prozesse am nichtlinearen Verhalten. D.h. durch geeignete Wahl der erregenden Wellenlänge sind noch weitaus höhere Werte der nichtlinearen Suszeptibilität zu erwarten. Für das Relaxationsverhalten des nichtlinearen Brechungsindexes bei entarteter Vierwellenmischung (DFWM) wurden die Relaxationskomponenten von 6 und 60 ps gemessen.

In den nachfolgenden Beispielen sind, falls jeweils nicht anders angegeben,

a) alle Mengenangaben auf das Gewicht bezogen;

b)alle Drücke 0,10 MPa (abs.);

c)alle Temperaturen 20° C .

In den Beispielen wurden die folgenden Verbindungen der Formel (3) hergestellt:

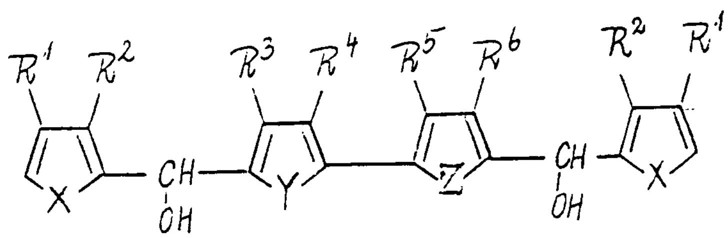

(3),

worin

**R³, R⁴, R⁵, R⁶** jeweils Wasserstoffatome bedeuten, **Y** und **Z** jeweils gleiche Reste sind und worin **R¹, R², X, Y** und **Z** die in der nachstehenden Tabelle 1 verzeichneten Bedeutungen haben:

Tabelle 1

| Verbindung | Beispiel Nr. | X | Y = Z | $R^1$ | $R^2$ |
|---|---|---|---|---|---|
| **A** | 1 | S | S | H | H |
| **B** | 2 | $N-CH_3$ | S | H | H |
| **C** | 3 | O | S | H | H |
| **D** | 8 | S | S | H | $CH_3$ |
| **E** | 4 | S | S | $CH_3$ | H |
| **F** | 5 | S | $N-CH_3$ | H | H |
| **G** | 6 | $N-CH_3$ | $N-CH_3$ | H | H |
| **H** | 7 | O | $N-CH_3$ | H | H |
| **J** | 11 | S | CH = CH | H | H |

## Beispiele

Herstellung von Verbindungen der Formel (1):

Beispiel 1: (Verbindung **A**)

Zu 2,75 g (32,7 mMol) wasserfreiem Thiophen in 25 ml absolutem Diethylether wurden unter Argonatmosphäre bei 0 °C 13,8 ml einer 2,15 molaren Lösung von Butyllithium zugetropft. Nach Zutropfende wurde die Kühlung entfernt und 20 Minuten unter Rückfluß gekocht. Anschließend wurden bei 0 °C 3 g 2,2'-Bithienyl-5,5'-dialdehyd in einer Suspension mit 100 ml wasserfreiem THF zugetropft. Nach 4,5-stündigem Rühren bei 0 °C wurde die Lösung 17 h bei 4 °C im Kühlschrank aufbewahrt. Nach Zusatz von 6,5 ml bei Raumtemperatur gesättigter Ammoniumchloridlösung und nachfolgendem Einwerfen von Trockeneis zur Neutralisation wurde von der wässrigen Phase und den Feststoffen abgetrennt. Nach Eindampfen des Lösungsmittels und Endtrocknen bei $10^{-3}$ mbar wurden 10,9g = 95 % d. Th. rohes IIIa erhalten. NMR-Spektrum in Aceton $D_6$: 7,40 ppm d, 2 H; 7,05 ppm d, 4 H; 6,97 ppm m, 2 H; 6,91 ppm d, 1 H; 6,30 ppm d, 1 H; 5,54 ppm d, 1 H.

Beispiel 2: (Verbindung **B**)

Zu einer Lösung von 3.65g (45 mMol) N-Methylpyrrol und 2.78g (23.9 mMol) Tetramethylethylendiamin (TMEDA) in 15 ml THF und 15 ml Ether wurden 16.2 ml (27.0 mMol) einer 1.59M n-Butyllithiumlösung bei Raumtemperatur zugegeben. Anschließend wurde 1.5 Stunden unter Rückfluß gekocht bis die Gasentwicklung (Butan) beendet war. Die Mischung wurde auf 0 °C abgekühlt und innerhalb von 30 Minuten eine Suspension von 3.0 g (13.5 mMol) 2,2'-Bithienyl-5,5'-dialdehyd in 50 ml THF zugegeben. Anschließend wurde 17 Stunden bei Raumtemperatur gerührt. Die Mischung wurde unter Eiskühlung in 60 ml einer gesättigten Ammoniumchloridlösung eingerührt. Die Lösung wurde mit 2 mal 100 ml Ether ausgeschüttelt und über Natriumsulfat getrocknet. Nach dem Abdampfen des Lösungsmittels im Wasserstrahlvakuum wurden 5.8 g eines schwarz-braun gefärbten, im Vakuum aufgeschäumten Feststoffes erhalten.

Beispiel 3: (Verbindung **C**)

Zu einer Lösung von 3.26 ml (45 mMol) Furan und 3.41 ml (27 mMol) TMEDA in 30 ml Ether wurden bei 0 °C 16.2 ml (27 mMol) einer 1.59 M n-Butyllithiumlösung so langsam zugetropft, daß die Temperatur nicht über 5 °C stieg. Nach beendeter Zugabe wurde die Mischung erwärmt und 2 Stunden unter Rückfluß gekocht. Der ausgefallene, hellbeige Niederschlag wurde durch Zugabe von 80 ml THF gelöst und die Mischung auf -14 °C abgekühlt. Bei dieser Temperatur wurde portionsweise 3.0 g (13.5 mMol) 2,2'-Bithienyl-5,5'-dialdehyd zugegeben. Die Mischung wurde langsam auf 15 °C erwärmt und bei dieser Temperatur 3.5 Stunden gerührt, danach in 200 ml einer Ether/Trockeneis-Mischung eingerührt. Nach dem Auftauen auf Raumtemperatur wurde mit 200 ml Wasser versetzt. Die Etherphase wurde abgetrennt, die wäßrige Phase mit Eisessig auf pH 3.5 eingestellt. Anschließend wurde die wäßrige Phase mit 3 mal 100 ml Ether ausgeschüttelt und die vereinigten Etherextrakte mit gesättigter Natriumcarbonatlösung neutralisiert und mit 2 mal 50 ml Wasser nachgewaschen. Die Etherfraktion wurde über Natriumsulfat getrocknet. Nach dem Abziehen des Lösungsmittels wurden 3.6 g = 74 % der Theorie eines braunen, hochviskosen Öls erhalten. H-NMR-Spektrum in Chloroform $D_1$: 7.4 ppm d, 2H; 6.97 ppm d, 2H; 6.86 ppm d, 2H; 6.33 ppm m, 4H; 6.33 ppm s, 2H; 3.08 ppm s(b), 2H.

Beispiel 4: (Verbindung **E**)

Analog zu Beispiel 1 wurden 2,14 g (21,8 mMol) 3-Methylthiophen, 2 g 2,2'-Bithienyl-5,5'-dialdehyd und 10,25 ml Butyllithiumlösung (1,93 Mol/l) umgesetzt. Nach gleicher Aufarbeitung wie im Beispiel 1 werden 3,7 g = 98 % der Theorie einer teils kristallinen teils glasigen Masse erhalten. Das H-NMR-Spektrum zeigt, daß das 3-Methylthiophen nicht eindeutig nur an der 5-Position mit der Aldehydgruppe reagierte, sondern daß 17 % der Thiopheneinheiten in der 2-Position mit der Aldehydfunktion zu Verbindung **D** reagiert haben. Das Hauptprodukt entspricht laut NMR-Spektrum jedoch dem gewünschten Produkt **E**. $^1$H-NMR-Spektrum in Aceton $D_6$: 7,05 ppm d, 2 H; 6,93 ppm m, 4 H; 6,63 ppm s, 2 H; 6,23 ppm s, 2 H; 5,48 ppm s, 2 H; 2,20 ppm s, 6 H.

Beispiel 5: (Verbindung **F**)

Eine Lösung von 5.0g (31.2 mMol) Bis-N-methylpyrrol und 9.4 ml (62.4 mMol) Tetramethylethylendiamin (TMEDA) in 70 ml Hexan wurde auf 0 °C abgekühlt. Zu dieser Mischung wurden 45.8 ml (68.6 mMol) einer 1.5 molaren n-Butyllithium-lösung in Hexan so langsam zugesetzt, daß die Temperatur nicht über 3 °C stieg. Nach beendeter Zugabe wurde für 30 Minuten unter Rückfluß gekocht. Die Lösung des bislithiierten Bis-N-methylpyrrols wurde zu einer auf 0 °C abgekühlten Lösung von 5.8 ml (62.4 mMol) Thiophen-2-carbaldehyd in 50 ml Ether so langsam zugetropft, daß die Temperatur nicht über 7 °C stieg. Nach beendeter Zugabe wurde auf Raumtemperatur erwärmt und 3 Stunden bei dieser Temperatur gerührt. Die Mischung wurde in 150 ml gesättigte Ammoniumchloridlösung unter Eiskühlung eingegossen. Die Mischung wurde 3 mal mit je 100 ml Ether ausgeschüttelt und die vereinigten Etherfraktionen wurden 2 mal

mit je 50 ml Wasser gewaschen. Nach dem Trocknen über Natriumsulfat wurde das Lösungsmittel im Wasserstrahlvakuum abgezogen, es wurden 7.5 g = 62.5 % der Theorie eines hellgelb-orangen Feststoffes erhalten, der nach etwa 2 Wochen Lagerung bei 4 °C verharzte. H-NMR-Spektrum in Chloroform $D_1$: 7.28 ppm d, 2H; 6.93 ppm m, 4H; 6.08 ppm m, 6H; 3.37 ppm s, 6H.

Beispiel 6: (Verbindung **G**)

Die Herstellung von dilithiiertem Bis-N-methylpyrrol erfolgte analog Beispiel 5. Dazu wurden 7.4 g (45.6 mMol) Bis-N-methylpyrrol, 11.6 ml (91.1 mMol) TMEDA und 62.9 ml (100.2 mMol) 1.59M n-Butyllithiumlösung verwendet. Nach dem Abkühlen auf Raumtemperatur wurde die Mischung zu einer Lösung von 9.8 ml (91.1 mMol) N-Methylpyrrol-2-carbaldehyd in 70 ml Diethylether bei 0 °C so langsam zugegeben, daß die Temperatur nicht über 4 °C stieg. Nach beendeter Zugabe wurde auf Raumtemperatur erwärmt und die Mischung 3 Stunden gerührt, dann wurde die Mischung auf 0 °C abgekühlt und 100 ml einer gesättigten Ammoniumchloridlösung zugetropft. Nach Zugabe von 150 ml Ether wurde die abgetrennte, wäßrige Phase noch 2 mal mit je 50 ml Ether extrahiert. Die vereinigten Etherextrakte wurden mit 2 mal 50 ml dest. Wasser gewaschen und über Natriumsulfat getrocknet. Nach Entfernen des Lösungsmittels im Vakuum wurden 13.9 g = 80.5 % der Theorie eines grün gefärbten Feststoffes erhalten. Zur Reinigung wurden 2 g dieses Produktes in 100 ml Triethylamin gelöst, vom Ungelösten abfiltriert und bei Raumtemperatur langsam mit 200 ml Hexan versetzt. Der ausgefallene weiße Niederschlag wurde abfiltriert und bei $10^{-3}$ mbar bei Raumtemperatur für 5 Stunden getrocknet. H-NMR in Chloroform $D_1$: 6.54, 6.06 ppm m, 12H; 3.62 ppm s, 2H; 3.34, 3.18 ppm m(b), 12H. Eine weitere Reinigung des Produktes durch Chromatographie war aufgrund der Sensibilität gegenüber Säuren nicht möglich.

Beispiel 7: (Verbindung **H**)

Analog Beispiel 6 wurden 5,0 g (30.8 mMol) dilithiiertes Bis-N-methylpyrrol mit 5.0 g (62.4 mMol) Furfural in 50 ml Ether bei 0 °C umgesetzt und aufgearbeitet.
Ausbeute: 9.8 g = 90.3 % der Theorie eines orange-roten, im Vakuum aufgeschäumten Feststoffes. H-NMR-Spektrum in Chloroform $D_1$: 7.43 ppm d, 2H; 6.34 ppm m, 4H; 6.08 ppm, m, 4H; 5.86 ppm s, 2H; 3.45 ppm s, 2H; 3.42 ppm s, 6H.

Beispiel 8: (Verbindung **D**)

6,30 g Dithienyl (37,9 mMol) in 25 ml absolutem Äther wurden mit 35,3 ml Butyllithium (75,8 mMol) in Hexan (2,15 Mol/l) bei 0 ° umgesetzt. Danach wurde auf Raumtemperatur erwärmt und 8,8 g (75,8 mMol) Tetramethylethylendiamin zugesetzt und eine Stunde unter Rückfluß erhitzt. Nach Abkühlen auf 0 ° wurden in 25 ml absolutem Äther 9,6 g (75,8 ml) 3-Methyl-2-thienylaldehyd (Hersteller Fa. Janssen, das Produkt enthält ca. 8 % 4-Methyl-2-thienylaldehyd) zugesetzt. Die Reaktionsmischung wurde 17 Stunden bei Raumtemperatur weitergerührt, dann mit 7 ml kalt gesättigter wäßriger $NH_4Cl$-Lösung und 15 ml Wasser hydrolisiert. Von dem abgesetzten schmierigen Rückstand wurde dekantiert und nach Abtrennen der Wasserschicht wurde die Ätherschicht zweimal mit je 10 ml Wasser extrahiert und danach im Vakuum eingedampft. Der Rückstand wurde dann zusammen mit dem vorher durch Abdekantieren isolierten Rückstand in 200 ml Isopropanol gelöst und filtriert. Nach Stehen über Nacht kristallisierten daraus 5,32 g = 37 % d. TH. an blaß-gelben Nadeln aus. Schmelzpunkt 182 °C. H-NMR in Aceton $D_6$: 7,02 ppm d, 2 H; 6,79 ppm d 2 H; 6,64 ppm d 2 H; 6,59 ppm d 2 H; 6,11 ppm s 1 H; 5,27 ppm s 2 H; 2,2 ppm s 6 H. Aus der eingedampften Isopropanol-Mutterlauge wurden 9,1 g eines zähen Harzes isoliert, das nach NMR-Untersuchungen, bedingt durch das im Ausgangsprodukt enthaltene 4-Methylisomer des Aldehyds, aus einem Isomerengemisch bestand.

Polykondensation von Verbindungen der Formel (1):

Beispiel 9:

1,48 g der gemäß Beispiel 1 herstellbaren Verbindung **A** wurden in 25 ml 1,2-Dichlorethan gelöst. Dazu wurden 72,4 mg p-Toluolsulfonsäuremonohydrat in 30 ml heißem 1,2-Dichlorethan zugefügt. Nach 4 1/2-stündigem Kochen unter Rückfluß wurde auf Raumtemperatur abgekühlt und 480 mg Jod in 12 ml 1,2-Dichlorethan bei 24 °C zugetropft. Nach Stehen über Nacht wurde der ausgefallene Niederschlag filtriert, fünfmal mit je 5 ml 1,2-Dichlorethan gewaschen und bei $10^{-3}$ mbar getrocknet. Ausbeute 1,87 g;

Leitfähigkeit $5 \cdot 10^{-5}$ S/cm.

Beschichtungen durch Polykondensation von Verbindungen der Formel (1) auf Substraten:

Beispiel 10:

150 mg Furfurylalkohol und 147 mg 2-Furaldehyd wurden in 8,3 ml einer 1 %igen PVC-Lösung in THF gelöst (PVC: Hersteller: Wacker-Chemie GmbH; Typenbezeichnung Y61 M, zur Nachreinigung aus THF mit Methanol umgefällt). Ein Teil dieser Lösung wurde mit einer Pipette bis zur vollständigen Bedeckung auf einen Objektträger aufgegeben. Danach ließ man das Lösungsmittel im Argonstrom verdampfen und legte danach den Objektträger in eine Petrischale, in die vorher 2 ml $POCl_3$ eingegeben worden waren, mit zwei Glasstäben als Abstandshalter ein. Nach 18 Stunden wurde der Objektträger herausgenommen, 5 Minuten in konzentrierte Salzsäure eingestellt, wobei sich die Beschichtung als Folie ablöste. Sie hatte nach der Trocknung bei Raumtemperatur eine Dicke von 20 $\mu$ und eine Leitfähigkeit von $1 \cdot 10^{-2}$ S/cm.

Herstellung von Polymerfilmen:

Beispiel 11:

Analog Beispiel 10 wurde zur Herstellung freistehender Filme in Polymermatrices eine Lösung des Diols zusammen mit PVC (Type wie Beispiel in 10) hergestellt. Die Anteile Monomer bezüglich PVC sind der Tabelle 2 zu entnehmen. Die Lösung wurde mit Hilfe eines Filmrakels (250 $\mu$m) auf eine Glasoberfläche aufgestrichen und durch Liegenlassen an der Luft für 2 bis 3 Stunden wurde das Lösungsmittel abgedampft. Aus dem so hergestellten, noch gequollenen Polymerfilm wurden Probekörper ($6 \times 2 cm^2$) ausgeschnitten und auf einen Objektträger gelegt. Die Folienstückchen wurden an beiden Enden mit Teflon-Isolierband auf dem Objektträger befestigt. Anschließend wurden die Proben auf einen Abstandshalter in eine Petrischale mit geschliffenem Rand eingelegt, in der sich jeweils 5 ml Phosphoroxychlorid bzw. 37 %-ige wäßrige Salzsäurelösung befanden.
Nach der in Tab. 2 aufgeführten Reaktionszeit wurden die Proben aus dem Gefäß genommen, 2 Stunden bei Raumtemperatur bei 1 mbar und dann 7 h bei $10^{-4}$ mbar getrocknet. Gegebenenfalls schloß sich eine Nachbehandlung an. Dabei wurden die Proben entweder in eine Ioddampfatmosphäre eingelegt oder die in Tabelle 2 angegebenen Zeiten in einen Trockenschrank der angegebenen Temperatur eingelegt. Im Falle der Ioddotierung wurde die Probe noch 2 Stunden bei 0.1 mbar evakuiert.

Tabelle 2: Polykondensation in weiteren Polymeren (Polymercomposites)

| Verbindung der Tabelle 1 | [mg] | PVC [mg] | Volumen der Lösung [ml] | Kondensationsmittel | Reaktionszeit [min] | Temperatur [°C] | Leitfähigkeit [S/cm] | Schichtdicke [µm] | |
|---|---|---|---|---|---|---|---|---|---|
| A | 215 | 700 | 10 | $POCl_3$ | 10 | 50 | $2,3*10^{-4}$ | 30 | |
| A | 215 | 700 | 10 | $POCl_3$ | 20 | 50 | $8,3*10^{-4}$ | 19 | |
| A | 215 | 700 | 10 | $POCl_3$ | 30 | 50 | $1,1*10^{-3}$ | 15 | |
| A | 215 | 700 | 10 | $POCl_3$ | 10 | 50 | $1,1*10^{-4}$ | 33 | 1 |
| F | 185 | 695 | 10 | HCl | 10 | 20 | $1,8*10^{-10}$ | 16 | |
| F | 185 | 695 | 10 | HCl | 10 | 20 | $6,0*10^{-10}$ | 20 | 2 |
| F | 185 | 695 | 10 | HCl | 10 | 20 | $1,5*10^{-10}$ | 19 | 3 |
| F | 185 | 695 | 10 | $POCl_3$ | 10 | 20 | $7,4*10^{-4}$ | 27 | |
| F | 185 | 695 | 10 | $POCl_3$ | 10 | 20 | $7,1*10^{-4}$ | 25 | 2 |
| F | 185 | 695 | 10 | $POCl_3$ | 10 | 20 | $7,5*10^{-4}$ | 21 | 3 |
| J | 210 | 700 | 10 | $POCl_3$ | 20 | 50 | $2,5*10^{-3}$ | 36 | 4 |
| J | 210 | 700 | 10 | $POCl_3$ | 10 | 50 | $2,7*10^{-4}$ | 17 | 4 |
| J | 210 | 700 | 10 | $POCl_3$ | 10 | 50 | $3,4*10^{-4}$ | 21 | 5 |
| G | 210 | 700 | 10 | HCl | 10 | 20 | $6,8*10^{-9}$ | 18 | |
| G | 210 | 700 | 10 | HCl | 10 | 20 | $1,6*10^{-9}$ | 16 | 6 |
| G | 210 | 700 | 10 | HCl | 10 | 20 | $1,7*10^{-9}$ | 23 | 2 |
| G | 210 | 700 | 10 | HCl | 10 | 20 | $7,4*10^{-8}$ | 12 | 8 |
| G | 210 | 700 | 10 | $POCl_3$ | 10 | 20 | $3,6*10^{-3}$ | 26 | |
| G | 210 | 700 | 10 | $POCl_3$ | 10 | 20 | $1,3*10^{-2}$ | 20 | 7 |
| G | 210 | 700 | 10 | $POCl_3$ | 10 | 20 | $2,6*10^{-3}$ | 36 | 2 |
| G | 210 | 700 | 10 | $POCl_3$ | 10 | 20 | $6,7*10^{-3}$ | 26 | 8 |

**Nachbehandlung der Proben:**

1 3 h Jod; 2 1 h/70 °C;
3 1 h/70 °C + 1 h Jod; 4 1 h/67 °C;
5 1 h/80 °C; 6 1 h Jod;
7 1 h Jod, 1 h 60 °C; 8 1 h/70 °C, 1 h Jod, 1 h/60 °C.

Beispiel 12: Beschichtungen mit Verbindungen der Formel (3)

Verbindungen der Formel (3) gemäß Tabelle 1 wurden mit einer Konzentration von 30 mg/ml in einer 1 %igen PVC-Lösung in THF (Type wie Beispiel 11) bzw. einer 1 %igen Poly-N-vinylcarbazollösung in THF

gelöst. Mit dieser Lösung wurden Glasscheiben von 7,6 cm (3") Durchmesser im Spincoating-Verfahren (700 - 1000 Umdrehungen pro Minute) beschichtet. Durch Überleiten eines Argonstroms wurde das Lösungsmittel abgedampft und mehrere beschichtete Scheiben auf einem Glasgestell in einem 1 l-Gefäß mit Planschliffdeckel eingestellt. Die Polykondensation der Verbindungen der Formel (3) wurde nach 4 verschiedenen Verfahren vorgenommen.

a) Einleiten von gasförmiger HCl über ein Glasrohr, das bis zum Boden des Gefässes reicht.

b) Einbringen von 2 ml eines flüssigen Kondensationsmittels auf den Boden des Reaktionsgefäßes und Evakuieren bei Raumtemperatur bis das Kondensationsmittel zum Sieden kam.

c) Evakuieren des Glasbehälters auf $10^{-3}$ mbar und Einströmenlassen des Kondensationsmitteldampfes aus einem Vorratsbehälter mit flüssigem Kondensationsmittel bis zum Sättigungsdampfdruck bei Raumtemperatur.

d) Evakuieren des Glasbehälters auf $10^{-3}$ mbar und Verdampfen von 10 $\mu$l eines flüssigen Kondensationsmittels aus einem 5 ml großen Vorratsbehälter, der während des Evakuierens mit flüssigem Stickstoff gekühlt war.

Bedingungen und Ergebnisse dieser Beschichtungen sind in der nachfolgenden Tabelle 3 aufgeführt.

Tabelle 3

| Herstellung und Eigenschaften von leitfähigen Beschichtungen nach Beispiel 12. | | | | | | |
|---|---|---|---|---|---|---|
| Verbindung (Tab.1) | x[a)] | Kondensationsmittel | Zeit [h] | Dicke [$\mu$] | Leitfähigkeit [S/cm] | $\epsilon^{b)}$ [$cm^{-1}$] |
| A | a | HCl | 120 | - | - | 6000 |
| A | b | $BF_3$ - Etherat | 24 | 0,35 | $3,70*10^{-8}$ | - |
| A | b | $CF_3COOH$ | 24 | 0,55 | $2,00*10^{-6}$ | 10400 |
| A | c | $CF_3COOH$ | 24 | 0,92 | $1,07*10^{-4}$ | 10100 |
| A | c | $CF_3COOH$[c)] | 24 | 0,96 | $1,40*10^{-8}$ | 9370 |
| A | c | $POCl_3$ | 24 | 1,70 | $8,50*10^{-4}$ | 4760 |
| A | d | $CF_3COOH$ | 24 | 0,72 | $5,00*10^{-9}$ | 9500 |
| G | a | HCl | 120 | - | - | 0 |
| G | b | $BF_3$ - Etherat | 24 | 0,35 | $4,50*10^{-8}$ | - |
| E | c | $POCl_3$ | 24 | 1,17 | $6,00*10^{-4}$ | 3960 |
| E | d | $CF_3COOH$ | 24 | 0,50 | $9,00*10^{-8}$ | 17000 |
| D | a | HCl | 120 | - | - | 3200 |
| D | b | $BF_3$ - Etherat | 24 | 0,35 | $2,80*10^{-8}$ | - |
| D | b | $CF_3COOH$ | 24 | 0,32 | $2,00*10^{-8}$ | 11300 |
| D | c | $CF_3COOH$ | 24 | 0,47 | $1,70*10^{-8}$ | 10900 |
| D | c | $CF_3COOH$[c)] | 24 | 0,62 | $1,00*10^{-8}$ | 7740 |
| D | c | $POCl_3$ | 24 | 0,92 | $3,00*10^{-5}$ | 3260 |
| D | d | $CF_3COOH$ | 24 | 0,40 | $1,30*10^{-8}$ | 11000 |

a) Kondensationsmethode
b) Extinktion am Maximum bei ca 1100 nm
c) Polyvinylcarbazol als Polymermatrix

Beispiel 13: Beschichtungen mit Verbindungen der Formel (3) auf Polymerfolien

Verbindungen der Formel (3) gemäß Tabelle 1 wurden in den in Tabelle 4 angegebenen Konzentrationen in Chloroform gelöst. Mit einem Rakel mit 250 $\mu$m Naßfilmstärke wurden die Lösungen auf Polymerfolien (Polyester: AMOTRANS R 40 Kopierfolie, Fa. Messerli AG, Schweiz; PVC hart: Wacker-Chemie) aufgetragen. Nach einer Trocknungszeit von 30 Minuten wurden Probekörper ($6*2cm^2$) ausgeschnitten und mit der beschichteten Seite nach oben mit einem Teflon-Isolierband befestigt.

Die Probenbehandlung erfolgte wie in Beispiel 11 beschrieben. Im Falle der Kondensation mit p-Toluolsulfonsäurelösung wurde der Probekörper in eine 10 %-ige wäßrige p-Toluolsulfonsäurelösung eingestellt.

Die mit dem Diol beschichteten Folien verfärbten sich bei Kontakt mit dem Kondensationsmittel sofort meist nach dunkelblau bzw. schwarz. Die Beschichtung war mechanisch stabil und kratzfest. In Abhängigkeit von

der Konzentration der Verbindung der Formel (3) in der Ausgangslösung konnten lichtdurchlässige, leitfähige Beschichtungen hergestellt werden.

Tabelle 4: Herstellung und Eigenschaften von leitfähigen Beschichtungen nach Beispiel 13

| Verbindung | Konz. d. Lösung [mg/ml] | Polymermatrix | Kond.-mittel | Zeit [min] | Oberflächen-Widerstand [Ohm] |
|---|---|---|---|---|---|
| G | 5 | Polyester | HCl | 10 | $4.5 \cdot 10^{11}$ |
| G | 5 | " | $POCl_3$ | 10 | $5.8 \cdot 10^{8}$ |
| G | 5 | " | p-TosH | 10 | $2.0 \cdot 10^{12}$ |
| G | 10 | " | HCl | 10 | $3.0 \cdot 10^{11}$ |
| G | 10 | " | p-TosH | 10 | $2.0 \cdot 10^{12}$ |
| G | 20 | " | HCl | 10 | $5.7 \cdot 10^{7}$ |
| G | 20 | " | $POCl_3$ | 10 | $4.1 \cdot 10^{8}$ |
| G | 20 | " | p-TosH | 10 | $9.7 \cdot 10^{7}$ |
| G | 5 | PVC, hart | HCl | 10 | $5.3 \cdot 10^{11}$ |
| G | 5 | " | $POCl_3$ | 10 | $7.8 \cdot 10^{11}$ |
| G | 20 | " | HCl | 10 | $1.2 \cdot 10^{9}$ |
| G | 20 | " | $POCl_3$ | 10 | $2.9 \cdot 10^{11}$ |
| G | 20 | " | p-TosH | 10 | $3.6 \cdot 10^{11}$ |
| A | 20[a)] | Polyester | HCl | 60 | $1.9 \cdot 10^{8}$ |
| A | 20[a)] | " | $POCl_3$ | 10 | $3.2 \cdot 10^{6}$ |
| A | 20[a)] | " | p-TosH | 10 | $2.0 \cdot 10^{12}$ |
| A | 20[b)] | PVC, hart | HCl | 60 | $1.0 \cdot 10^{12}$ |
| A | 20[b)] | " | HCl | 300 | $5.0 \cdot 10^{11}$ |
| A | 20[b)] | " | $POCl_3$ | 10 | $8.2 \cdot 10^{6}$ |
| A | 20[b)] | " | p-TosH | 10 | $5.2 \cdot 10^{11}$ |
| F | 20 | Polyester | HCl | 10 | $4.0 \cdot 10^{11}$ |
| F | 20 | " | $POCl_3$ | 10 | $9.0 \cdot 10^{9}$ |
| F | 20 | " | p-TosH | 10 | $4.0 \cdot 10^{11}$ |

a) Lösungsmittel : THF

b) Lösungsmittel : $THF/CHCl_3$ = 1 : 4 Volumenteile.

**Patentansprüche**
**Patentansprüche für folgende Vertragsstaaten : AT, BE, CH, DE, FR, GB, IT, LI, NL**

1. Verbindungen der Formel (1)

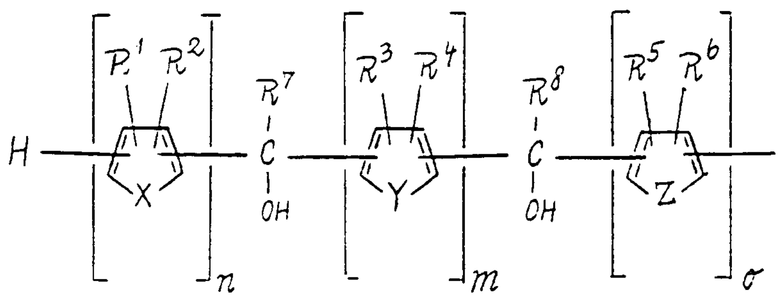

(1),

worin bedeuten

| | |
|---|---|
| **$R^1$**, **$R^2$**, **$R^3$**, **$R^4$**, **$R^5$** und **$R^6$** | gleiche oder verschiedene ringständige Reste, nämlich Wasserstoffatome, Halogenatome, geradkettige oder verzweigte $C_1$- bis $C_6$-Alkylreste, $C_1$-bis $C_6$-Alkylcarbonsäurereste oder deren Ester mit $C_1$-bis $C_4$-Alkanolen, $C_1$- bis $C_6$-Alkylaminoreste, Nitro- oder Cyanogruppen, wobei mindestens zwei der Reste **$R^1$**, **$R^2$**, **$R^3$**, **$R^4$**, **$R^5$** und **$R^6$** paarweise miteinander verknüpft sein können und eine Trimethylen- oder Tetramethylengruppe ausbilden oder mit dem Ring, an dem sie gebunden sind einen mehrkernigen (Hetero)aromaten bilden; |
| **X**, **Y** und **Z** | gleiche oder verschiedene zweiwertige Reste -O-, -S-, -N($R^9$)- oder -C($R^{10}$)=C($R^{11}$)-, unter der Bedingung, daß mindestens einer der Reste X, Y und Z kein Rest der Formel -C($R^{10}$)=C($R^{11}$)- ist, und worin |
| **$R^9$** | ein Wasserstoffatom, ein verzweigter oder unverzweigter $C_1$-bis $C_8$-Alkylrest oder ein gegebenenfalls substituierter Phenylrest ist, |
| **$R^{10}$** und **$R^{11}$** | jeweils gleiche oder verschiedene Reste sind, welche eine der Bedeutungen von $R^1$ haben, |
| **$R^7$** und **$R^8$** | gleiche oder verschiedene Reste, nämlich Wasserstoffatome, $C_1$- bis $C_6$-Alkyl- oder Phenylreste, |
| **n**,**m** und **o** | gleiche oder verschieden ganze Zahlen im Wert von 1 bis 10. |

2. Verbindungen nach Anspruch 1, welche die Formel (2)

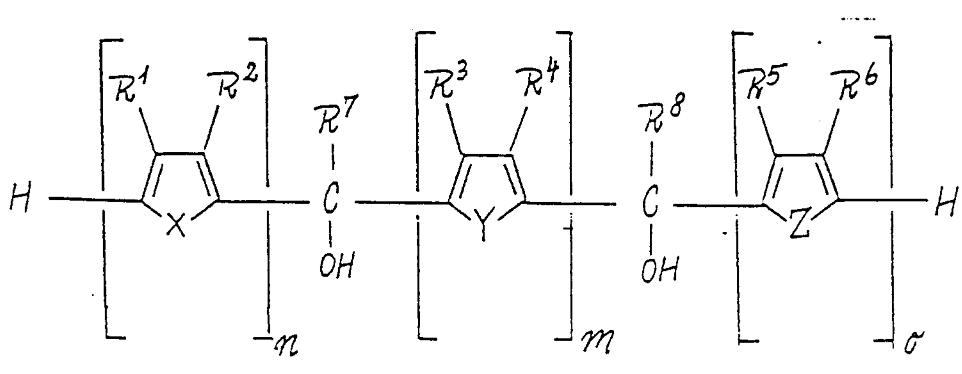

(2),

aufweisen,
worin **$R^1$**, **$R^2$**, **$R^3$**, **$R^4$**, **$R^5$**, **$R^6$**, **$R^7$**, **$R^8$**, **X**, **Y**, **Z**, **m**, **n** und **o** die in Anspruch 1 angegebenen Bedeutungen haben.

**3.** Verbindungen nach Anspruch 1 oder 2, welche die Formel (3)

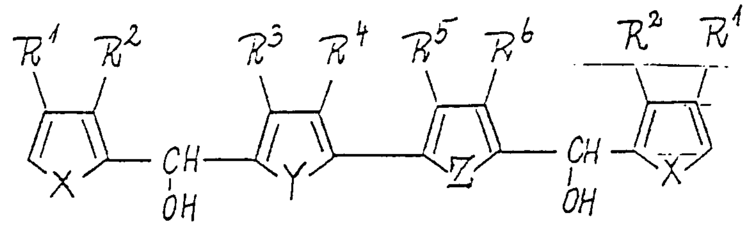

(3),

aufweisen,
worin **X**, **Y**, **Z**, **R¹**, **R²**, **R³**, **R⁴**, **R⁵** und **R⁶** die in Anspruch 1 angegebene Bedeutung haben.

**4.** Verfahren zur Herstellung von Verbindungen der Formel (1) durch Umsetzung von Verbindungen der Formel (4) mit Verbindungen der Formeln (5) und (6)

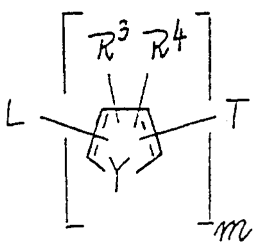

(4),

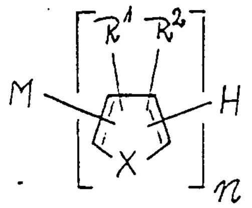

(5),

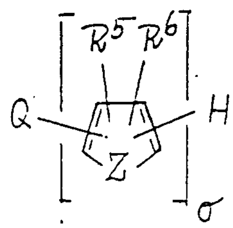

(6),

worin **entweder**
beide Reste **L** und **T** Reste aus der Gruppe der Wasserstoffatome, Metallatome und Reste der Formel -MgI, -MgBr und -MgCl sind und

**M** ein Rest der Formel $-C(R^7)=O$ und
**Q** ein Rest der Formel $-C(R^8)=O$ ist
**oder**
beide Reste **M** und **Q** Reste aus der Gruppe der Wasserstoffatome, Metallatome und Reste der Formel -MgI, -MgBr und -MgCl sind und
**L** ein Rest der Formel $-C(R^7)=O$ und
**T** ein Rest der Formel $-C(R^8)=O$ ist und
worin **$R^1$**, **$R^2$**, **$R^3$**, **$R^4$**, **$R^5$**, **$R^6$**, **$R^7$**, **$R^8$**, **X**, **Y**, **Z**, **m**, **n** und **o** die in Anspruch 1 angegebenen Bedeutungen haben.

**5.** Verfahren zur Herstellung von Polymeren mit (Hetero-)arylenmethineinheiten durch Polykondensation von Verbindungen gemäß Anspruch 1, 2 oder 3 in Gegenwart von Kondensationsmittel, welches ausgewählt wird aus der Gruppe der Säurechloride, Säureanhydride, starken Säuren, Basen und hygroskopischen Verbindungen.

**6.** Verfahren nach Anspruch 5, wobei vor, während oder nach der Polykondensation Dotierungsmittel zugegeben wird, welches ausgewählt wird aus der Gruppe der Alkalimetalle, Protonensäuren und Lewis-Säuren.

**Patentansprüche für folgenden Vertragsstaat : ES**

1. Verfahren zur Herstellung von Verbindungen der Formel (1)

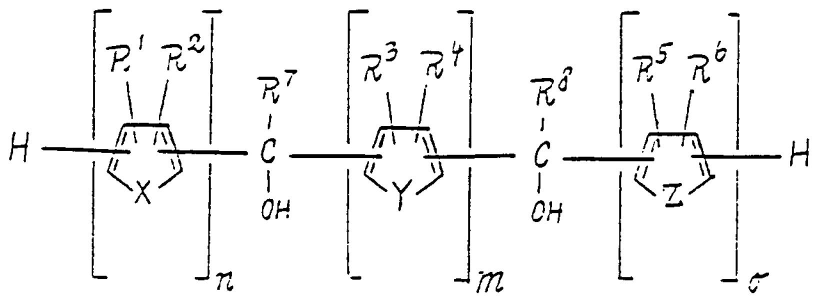

(1),

worin bedeuten

**$R^1$**, **$R^2$**, **$R^3$**, **$R^4$**, **$R^5$** und **$R^6$** gleiche oder verschiedene ringständige Reste, nämlich Wasserstoffatome, Halogenatome, geradkettige oder verzweigte $C_1$- bis $C_6$-Alkylreste, $C_1$-bis $C_6$-Alkylcarbonsäurereste oder deren Ester mit $C_1$-bis $C_4$-Alkanolen, $C_1$- bis $C_6$-Alkylaminoreste, Nitro- oder Cyanogruppen, wobei mindestens zwei der Reste **$R^1$**, **$R^2$**, **$R^3$**, **$R^4$**, **$R^5$** und **$R^6$** paarweise miteinander verknüpft sein können und eine Trimethylen- oder Tetramethylengruppe ausbilden oder mit dem Ring, an dem sie gebunden sind einen mehrkernigen (Hetero)aromaten bilden;

**X**, **Y** und **Z** gleiche oder verschiedene zweiwertige Reste -O-, -S-, -N($R^9$)- oder -C($R^{10}$)=C($R^{11}$)-, unter der Bedingung, daß mindestens einer der Reste X, Y und Z kein Rest der Formel -C($R^{10}$)=C($R^{11}$)- ist, und worin

**$R^9$** ein Wasserstoffatom, ein verzweigter oder unverzweigter $C_1$-bis $C_8$-Alkylrest oder ein gegebenenfalls substituierter Phenylrest ist,

**$R^{10}$** und **$R^{11}$** jeweils gleiche oder verschiedene Reste sind, welche eine der Bedeutungen von $R^1$ haben,

**$R^7$** und **$R^8$** gleiche oder verschiedene Reste, nämlich Wasserstoffatome, $C_1$- bis $C_6$-Alkyl- oder Phenylreste,

**n**,**m** und **o** gleiche oder verschieden ganze Zahlen im Wert von 1 bis 10, durch Umsetzung von Verbindungen der Formel (4) mit Verbindungen der Formeln (5) und (6)

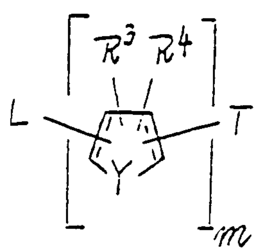

(4),

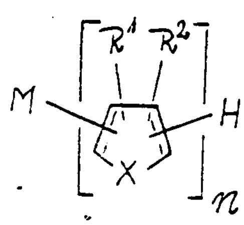

(5),

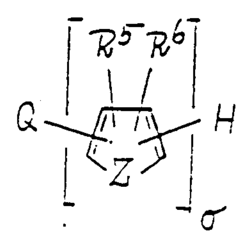

(6),

worin **entweder**

beide Reste **L** und **T** Reste aus der Gruppe der Wasserstoffatome, Metallatome und Reste der Formel -MgI, -MgBr und -MgCl sind und

**M** ein Rest der Formel -C($R^7$)=O und

**Q** ein Rest der Formel -C($R^8$)=O ist

**oder**

beide Reste **M** und **Q** Reste aus der Gruppe der Wasserstoffatome, Metallatome und Reste der Formel -MgI, -MgBr und -MgCl sind und

| | |
|---|---|
| **L** | ein Rest der Formel -C($R^7$) = O und |
| **T** | ein Rest der Formel -C($R^8$) = O ist und |
| | worin **$R^1$**, **$R^2$**, **$R^3$**, **$R^4$**, **$R^5$**, **$R^6$**, **$R^7$**, **$R^8$**, **X**, **Y**, **Z**, **m**, **n** und **o** die vorstehenden Bedeutungen haben. |

**2.** Verfahren nach Anspruch 1 zur Herstellung von Verbindungen der Formel (2)

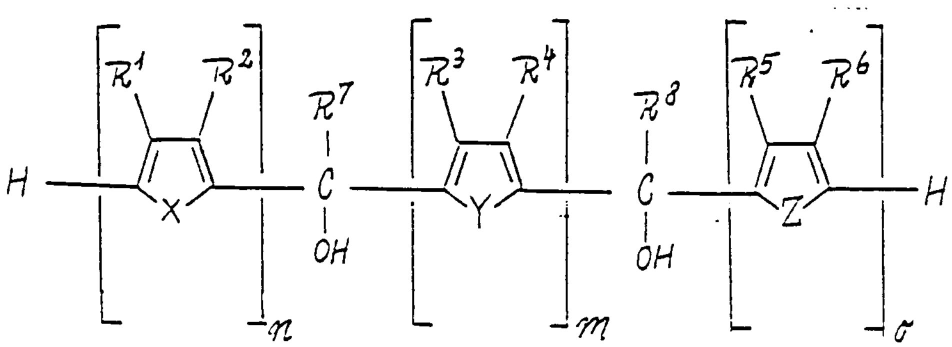

(2),

worin **$R^1$, $R^2$, $R^3$, $R^4$, $R^5$, $R^6$, $R^7$, $R^8$, X, Y, Z, m, n** und **o** die in Anspruch 1 angegebenen Bedeutungen haben.

**3.** Verfahren nach Anspruch 1 zur Herstellung von Verbindungen der Formel (3)

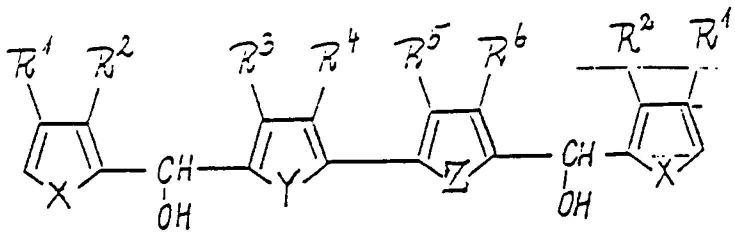

(3),

worin **X,Y, Z, $R^1$, $R^2$, $R^3$, $R^4$, $R^5$** und **$R^6$** die in Anspruch 1 angegebene Bedeutung haben.

**4.** Verfahren zur Herstellung von Polymeren mit (Hetero-)arylenmethineinheiten durch Polykondensation von gemäß Anspruch 1, 2 oder 3 hergestellten Verbindungen, in Gegenwart von Kondensationsmittel, welches ausgewählt wird aus der Gruppe der Säurechloride, Säureanhydride, starken Säuren, Basen und hygroskopischen Verbindungen.

**5.** Verfahren nach Anspruch 4, wobei vor, während oder nach der Polykondensation Dotierungsmittel zugegeben wird, welches ausgewählt wird aus der Gruppe der Alkalimetalle, Protonensäuren und Lewis-Säuren.

**6.** Verfahren nach Anspruch 4 durch Polykondensation von Verbindungen der Formel (1) zu Polymeren, welche Einheiten der Formel (7) enthalten

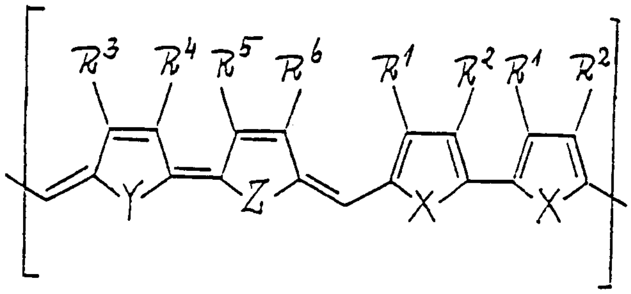

(7),

worin **$R^1$, $R^2$, $R^3$, $R^4$, $R^5$, $R^6$, X, Y, Z** die in Anspruch 1 angegebenen Bedeutungen haben, mit der Maßgabe, daß mindestens einer der gemäß Formel (7) durch eine Gruppe der Formel =CH- verbundenen Ringe sich durch mindestens einen der Reste **$R^1$, $R^2$, $R^3$, $R^4$, $R^5$, $R^6$, X, Y, Z** von den übrigen unterscheidet.

**7.** Verfahren gemäß Anspruch 6, wobei vor, während oder nach der Polykondensation Dotierungsmittel zugegeben wird, welches ausgewählt wird aus der Gruppe der Alkalimetalle, Protonensäuren und Lewis-Säuren.

**Claims**

**Claims for the following Contracting States : AT, BE, CH, DE, FR, GB, IT, LI, NL**

**1.** Compounds of the formula (1)

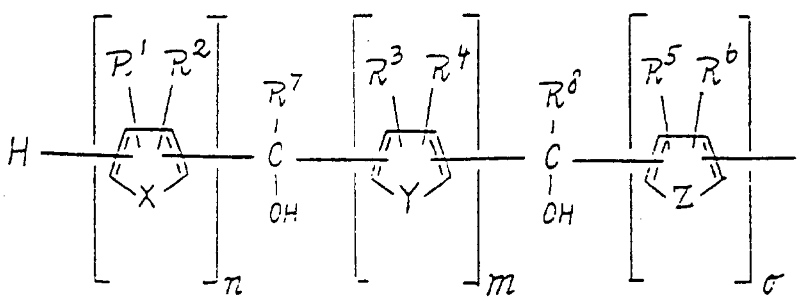

(1)

wherein

| | |
|---|---|
| $R^1$, $R^2$, $R^3$, $R^4$, $R^5$ and $R^6$ | denote identical or different radicals on the rings, that is to say hydrogen atoms, halogen atoms, straight-chain or branched $C_1$-to $C_6$-alkyl radicals, $C_1$- to $C_6$-alkylcarboxylic acid radicals or esters thereof with $C_1$- to $C_4$-alkanols, $C_1$- to $C_6$-alkylamino radicals or nitro or cyano groups, wherein at least two of the radicals $R^1$, $R^2$, $R^3$, $R^4$, $R^5$ and $R^6$ can be linked to one another in pairs and form a trimethylene or tetramethylene group or form a polynuclear (hetero)aromatic with the ring to which they are bonded; |
| X, Y and Z | denote identical or different divalent radicals -O-, -S-, -N($R^9$)- or -C-($R^{10}$)=C($R^{11}$)-, with the proviso that at least one of the radicals X, Y and Z is not a radical of the formula -C($R^{10}$)=C($R^{11}$)-, and wherein |
| $R^9$ | is a hydrogen atom, a branched or unbranched $C_1$- to $C_8$-alkyl radical or an optionally substituted phenyl radical and |
| $R^{10}$ and $R^{11}$ | are in each case identical or different radicals which have one of the meanings of $R^1$, |
| $R^7$ and $R^8$ | denote identical or different radicals, that is to say hydrogen atoms or $C_1$- to $C_6$-alkyl or phenyl radicals, and |
| n, m and o | denote identical or different integers with a value from 1 to 10. |

2. Compounds according to Claim 1 which have the formula (2)

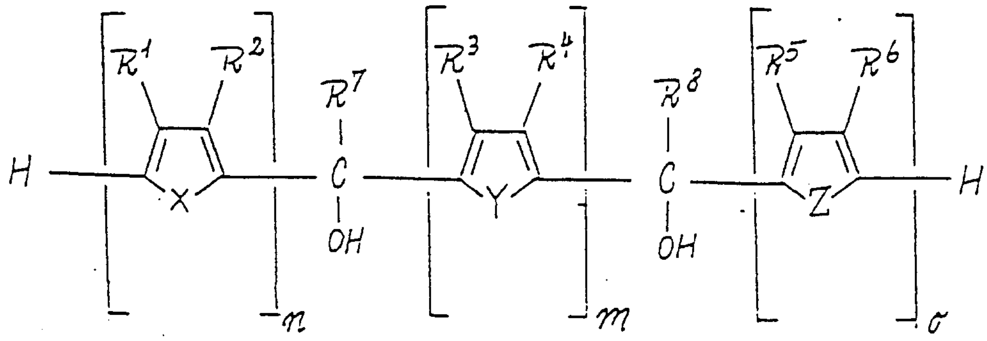

(2)

wherein $R^1$, $R^2$, $R^3$, $R^4$, $R^5$, $R^6$, $R^7$, $R^8$, X, Y, Z, m, n and o have the meanings given in Claim 1.

3. Compounds according to Claim 1 or 2 which have the formula (3)

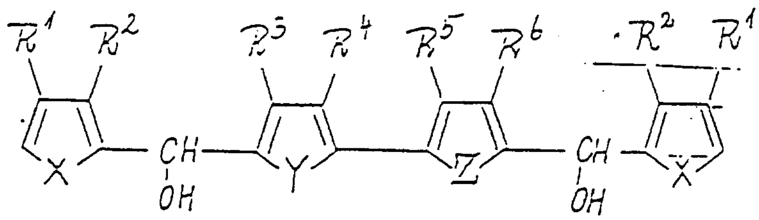

(3)

wherein X, Y, Z, $R^1$, $R^2$, $R^3$, $R^4$, $R^5$ and $R^6$ have the meaning given in Claim 1.

4. Process for the preparation of compounds of the formula (1) by reaction of compounds of the formula (4) with compounds of the formulae (5) and (6)

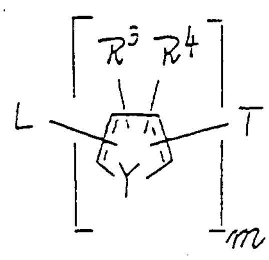

(4),

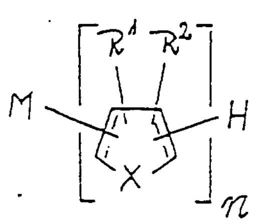

(5),

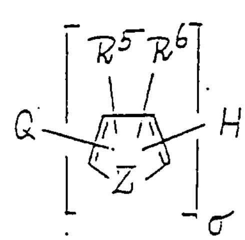

(6),

wherein either

the two radicals L and T are radicals from the group comprising hydrogen atoms, metal atoms and radicals of the formula -MgI, -MgBr and -MgCl and

- M is a radical of the formula $-C(R^7)=O$ and
- Q is a radical of the formula $-C(R^8)=O$, or the two radicals M and Q are radicals from the group comprising hydrogen atoms, metal atoms and radicals of the formula -MgI, -MgBr and -MgCl and
- L is a radical of the formula $-C(R^7)=O$ and
- T is a radical of the formula $-C(R^8)=O$, and wherein $R^1$, $R^2$, $R^3$, $R^4$, $R^5$, $R^6$, $R^7$, $R^8$, X, Y, Z, m, n and o have the meanings given in Claim 1.

5. Process for the preparation of polymers with (hetero-)arylenemethine units by polycondensation of compounds according to any one of Claims 1, 2 and 3 in the presence of a condensing agent which is

selected from the group comprising acid chlorides, acid anhydrides, strong acids, bases and hygroscopic compounds.

**6.** Process according to Claim 5, wherein a doping agent is added before, during or after the polycondensation which is selected from the group comprising the alkali metals, protonic acids and Lewis acids.

**Claims for the following Contracting State : ES**

**1.** Process for the preparation of compounds of the formula (1)

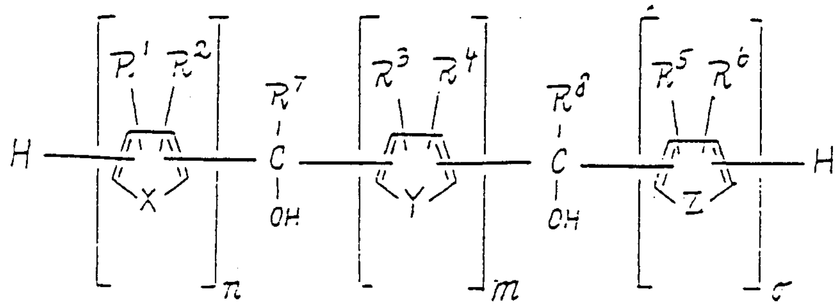

(1)

wherein

| | |
|---|---|
| $R^1$, $R^2$, $R^3$, $R^4$, $R^5$ and $R^6$ | denote identical or different radicals on the rings, that is to say hydrogen atoms, halogen atoms, straight-chain or branched $C_1$-to $C_6$-alkyl radicals, $C_1$- to $C_6$-alkylcarboxylic acid radicals or esters thereof with $C_1$- to $C_4$-alkanols, $C_1$- to $C_6$-alkylamino radicals or nitro or cyano groups, wherein at least two of the radicals $R^1$, $R^2$, $R^3$, $R^4$, $R^5$ and $R^6$ can be linked to one another in pairs and form a trimethylene or tetramethylene group or form a polynuclear (hetero)aromatic with the ring to which they are bonded; |
| X, Y and Z | denote identical or different divalent radicals -O-, -S-, -N($R^9$)- or -C-($R^{10}$)=C($R^{11}$)-, with the proviso that at least one of the radicals X, Y and Z is not a radical of the formula -C($R^{10}$)=C($R^{11}$)-, and wherein |
| $R^9$ | is a hydrogen atom, a branched or unbranched $C_1$- to $C_8$-alkyl radical or an optionally substituted phenyl radical and |
| $R^{10}$ and $R^{11}$ | are in each case identical or different radicals which have one of the meanings of $R^1$, |
| $R^7$ and $R^8$ | denote identical or different radicals, that is to say hydrogen atoms or $C_1$- to $C_6$-alkyl or phenyl radicals, and |
| n, m and o | denote identical or different integers with a value from 1 to 10, |

by reaction of compounds of the formula (4) with compounds of the formulae (5) and (6)

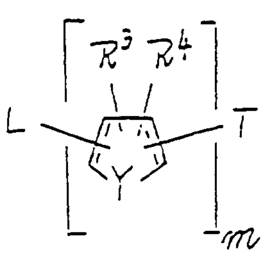

(4),

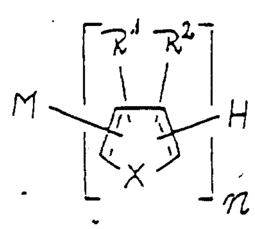

(5),

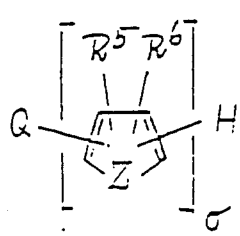

(6),

wherein either
the two radicals L and T are radicals from the group comprising hydrogen atoms, metal atoms and radicals of the formula -MgI, -MgBr

and -MgCl and

M is a radical of the formula -C($R^7$) = O and

Q is a radical of the formula -C($R^8$) = O,

or

the two radicals M and Q are radicals from the group comprising hydrogen atoms, metal atoms and radicals of the formula -MgI, -MgBr and -MgCl and

L is a radical of the formula -C($R^7$) = O and

T is a radical of the formula -C($R^8$) = O, and

wherein $R^1$, $R^2$, $R^3$, $R^4$, $R^5$, $R^6$, $R^7$, $R^8$, X, Y, Z, m, n and o have the aforementioned meanings.

2. Process according to Claim 1 for the preparation of compounds of the formula (2)

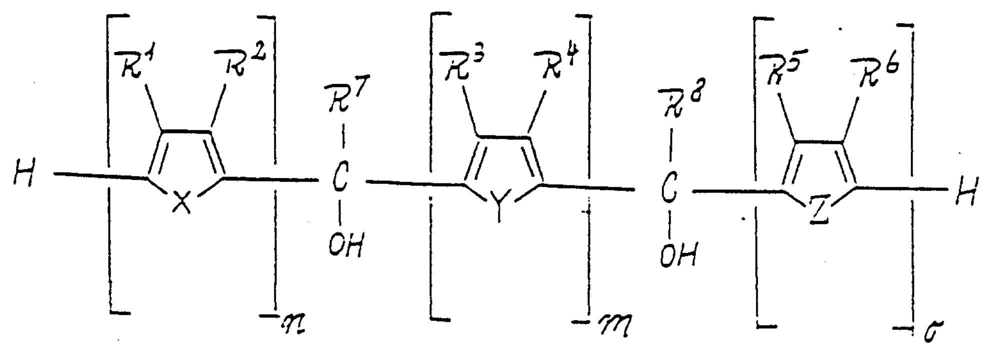

(2)

wherein $R^1$, $R^2$, $R^3$, $R^4$, $R^5$, $R^6$, $R^7$, $R^8$, X, Y, Z, m, n and o have the meanings given in Claim 1.

3. Process according to Claim 1 for the preparation of compounds of the formula (3)

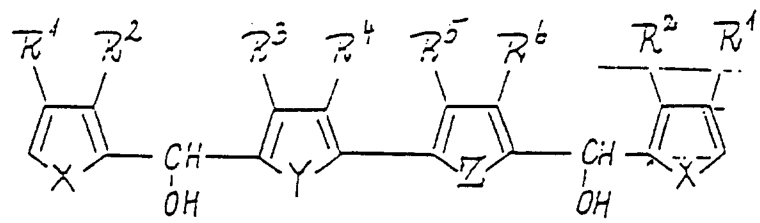

(3)

wherein X, Y, Z, $R^1$, $R^2$, $R^3$, $R^4$, $R^5$ and $R^6$ have the meaning given in Claim 1.

4. Process for the preparation of polymers with (hetero-)arylenemethine units by polycondensation of compounds prepared according to Claim 1, 2 or 3 in the presence of a condensing agent which is selected from the group comprising acid chlorides, acid anhydrides, strong acids, bases and hygroscopic compounds.

5. Process according to Claim 4, wherein a doping agent is added before, during or after the polycondensation which is selected from the group comprising the alkali metals, protonic acids and Lewis acids.

6. Process according to Claim 4 by polycondensation of compounds of the formula (1) to polymers which contain units of the formula (7)

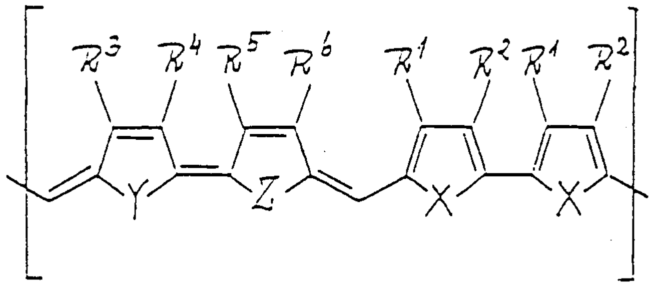

(7)

wherein $R^1$, $R^2$, $R^3$, $R^4$, $R^5$, $R^6$, X, Y and Z have the meanings given in Claim 1, with the proviso that at least one of the rings according to formula (7) bonded by a group of the formula =CH- differs from the others by at least one of the radicals $R^1$, $R^2$, $R^3$, $R^4$, $R^5$, $R^6$, X, Y and Z.

**7.** Process according to Claim 6, wherein a doping agent is added before, during or after the polycondensation which is selected from the group comprising the alkali metals, protonic acids and Lewis acids.

## Revendications

**Revendications pour les Etats contractants suivants : AT, BE, CH, DE, FR, GB, IT, LI, NL**

**1.** Composés de formule (1)

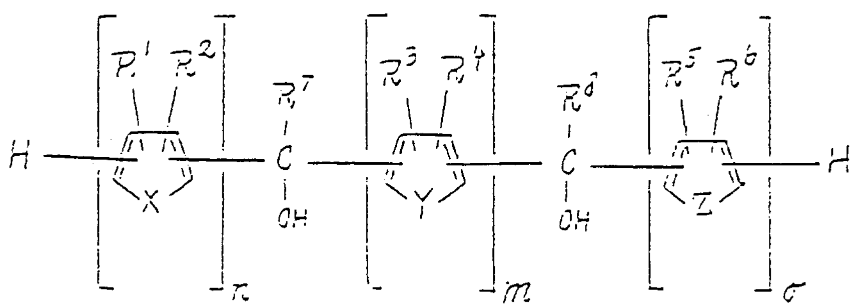

(1),

où

$R^1$, $R^2$, $R^3$, $R^4$, $R^5$ et $R^6$ représentent des radicaux identiques ou différents sur les noyaux, c'est-à-dire des atomes d'hydrogène, des atomes d'halogènes, des radicaux alkyles en $C_1$ à $C_6$ à chaînes droites ou ramifiées, des radicaux d'acides carboxyliques d'alkyles en $C_1$ à $C_6$ ou de leurs esters avec des alcanols en $C_1$ à $C_4$, des radicaux amino d'alkyles en $C_1$ à $C_6$,des radicaux nitro ou cyano, au moins deux des radicaux $R^1$, $R^2$, $R^3$, $R^4$, $R^5$ et $R^6$ pouvant être reliés entre eux par paires et formant un groupe triméthylène ou un groupe tétraméthylène ou formant avec le noyau auquel ils sont reliés un (hétéro)aromatique à plusieurs noyaux ;

X, Y et Z représentent les radicaux bivalents identiques ou différents -O-, -S-, -N($R^9$)- ou -C($R^{10}$)≡C-($R^{11}$)-, à la condition qu'au moins un des radicaux X, Y et Z ne soit pas un radical de formule -C($R^{10}$)-=C($R^{11}$)-, et ou

$R^9$ représente un atome d'hydrogène, un radical alkyle en $C_1$ à $C_8$ ramifié ou non ramifié ou un radical phényle éventuellement substitué,

$R^{10}$ et $R^{11}$ représentent chacun des radicaux identiques ou différentsqui ont une des significations de $R^1$,

$R^7$ et $R^8$ représentent des radicaux identiques ou différents c'est-à-dire des atomes d'hydrogène, des radicaux alkyles en $C_1$ à $C_6$ ou des radicaux phényle,

n, m et o représentent des nombres entiers identiques ou différents dont la valeur va de 1 à 10.

**2.** Composés selon la revendication 1, qui présentent la formule (2)

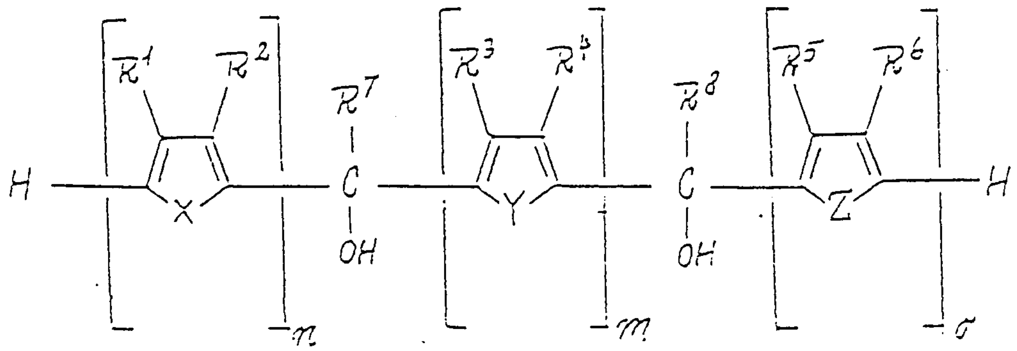

(2),

où $R^1$, $R^2$, $R^3$, $R^4$, $R^5$, $R^6$, $R^7$, $R^8$, X, Y, Z, m, n et o ont les significations données dans la revendication 1.

**3.** Composés selon la revendication 1 ou 2, qui présentent la formule (3)

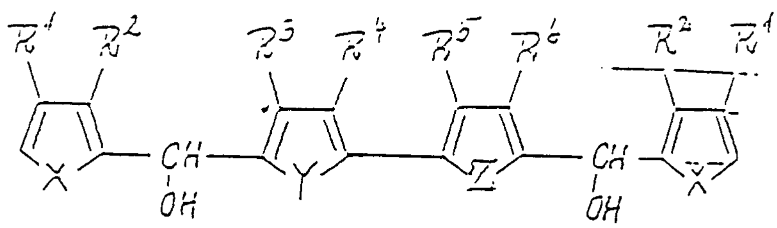

(3),

où X, Y, Z, $R^1$, $R^2$, $R^3$, $R^4$, $R^5$ et $R^6$ ont les significations données dans la revendication 1.

**4.** Procédé pour préparer des composés de formule (1) par réaction des composés de formule (4) avec des composés des formules (5) et (6)

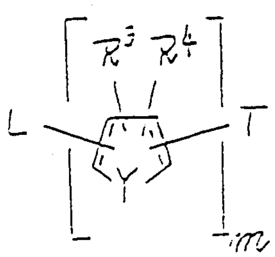

(4),

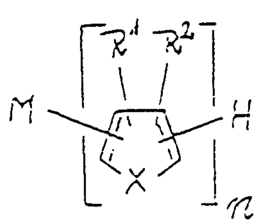

(5),

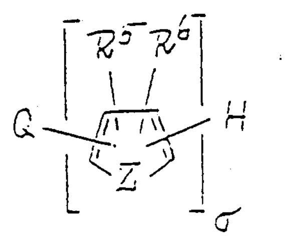

(6),

où, soit
les deux radicaux L et T sont des radicaux du groupe des atomes d'hydrogène, des atomes métalliques et des radicaux de formule -MgI, -MgBr et -MgCl, et
M est un radical de formule $-C(R^7)=O$ et
Q est un radical de formule $-C(R^8)=O$,
soit
les deux radicaux M et Q sont des radicaux du groupe des atomes d'hydrogène, des atomes métalliques et des radicaux de formule -MgI, -MgBr et -MgCl, et
L est un radical de formule$-C(R^7)=O$, et

T est un radical de formule -C($R^8$) = O, et
où $R^1$, $R^2$, $R^3$, $R^4$, $R^5$, $R^6$, $R^7$, $R^8$, X, Y, Z, m, n et o ont les significations données dans la revendication 1.

**5.** Procédé pour préparer des polymères ayant des unités (hétéro-)arylèneméthines par polycondensation des composés selon la revendication 1, 2 ou 3 en présence d'un agent de condensation choisi dans le groupe des chlorures d'acides, des anhydrides d'acides, des acides forts, des bases et des composés hygroscopiques.

**6.** Procédé selon la revendication 5, où l'on ajoute un agent de dopage avant, pendant ou après la polycondensation, cet agent étant choisi dans le groupe des métaux alcalins, des acides protoniques et des acides de Lewis.

**Revendications pour l'Etat contractant suivant : ES**

**1.** Procédé pour préparer des composés de formule (1)

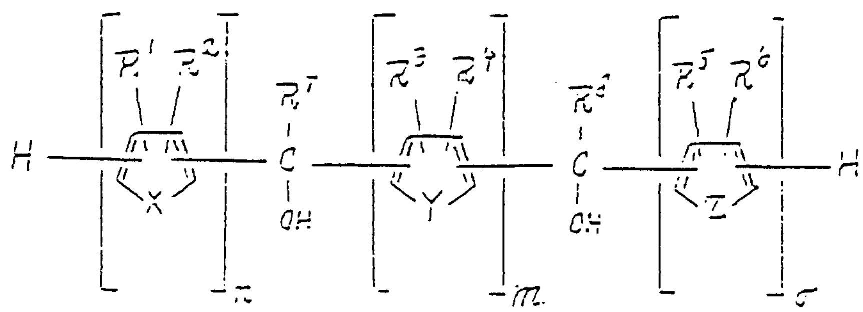

(1),

où
$R^1$, $R^2$, $R^3$, $R^4$, $R^5$ et $R^6$ représentent des radicaux identiques ou différents sur les noyaux, c'est-à-dire des atomes d'hydrogène, des atomes d'halogènes, des radicaux alkyles en $C_1$ à $C_6$ à chaînes droites ou ramifiées, des radicaux d'acides carboxyliques d'alkyles en $C_1$ à $C_6$ ou de leurs esters avec des alcanols en $C_1$ à $C_4$, des radicaux amino d'alkyles en $C_1$ à $C_6$, des radicaux nitro ou cyano, au moins deux des radicaux $R^1$, $R^2$, $R^3$, $R^4$, $R^5$ et $R^6$ pouvant être reliés entre eux par paires et formant un groupe triméthylène ou un groupe tétraméthylène ou formant avec le noyau auquel ils sont reliés un (hétéro)aromatique à plusieurs noyaux ;
X, Y et Z représentent les radicaux bivalents identiques ou différents -O-, -S-, -N($R^9$)- ou -C($R^{10}$)≡C-($R^{11}$)-, à la condition qu'au moins un des radicaux X, Y et Z ne soit pas un radical de formule -C($R^{10}$)-= C($R^{11}$)-, et où
$R^9$ représente un atome d'hydrogène, un radical alkyle en $C_1$ à $C_8$ ramifié ou non ramifié ou un radical phényle éventuellement substitué,
$R^{10}$ et $R^{11}$ représentent chacun des radicaux identiques ou différentsqui ont une des significations de $R^1$,
$R^7$ et $R^8$ représentent des radicaux identiques ou différents , c'est-à-dire des atomes d'hydrogène, des radicaux alkyles en $C_1$ à $C_6$ ou des radicaux phényle,
n, m et o représentent des nombres entiers identiques ou différents dont la valeur va de 1 à 10,
par réaction des composés de formule (4) avec des composés des formules (5) et (6)

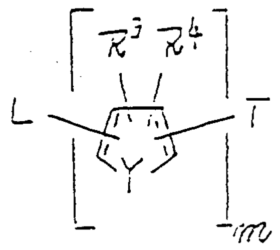

(4) ,

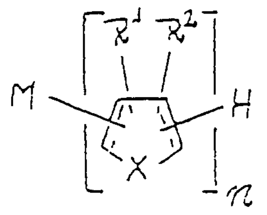

(5) ,

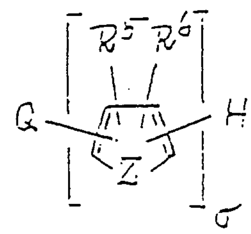

(6) ,

où, soit
les deux radicaux L et T sont des radicaux du groupe des atomes d'hydrogène, des atomes métalliques et des radicaux de formule -MgI, -MgBr et -MgCl, et
M est un radical de formule $-C(R^7)=O$ et
Q est un radical de formule $-C(R^8)=O$,
soit
les deux radicaux M et Q sont des radicaux du groupe des atomes d'hydrogène, des atomes métalliques et des radicaux de formule -MgI, -MgBr et -MgCl, et
L est un radical de formule$-C(R^7)=O$, et
T est un radical de formule $-C(R^8)=O$, et
où $R^1$, $R^2$, $R^3$, $R^4$, $R^5$, $R^6$, $R^7$, $R^8$, X, Y, Z, m, n et o ont les significations précédentes.

**2.** Procédé selon la revendication 1 pour préparer des composés de formule (2)

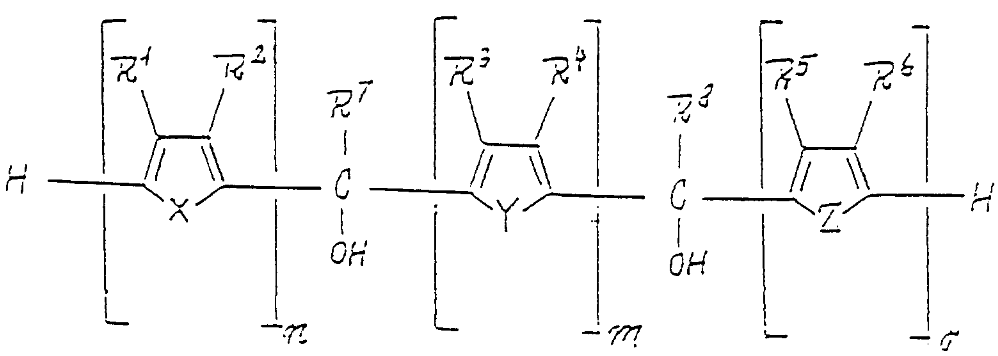

(2) ,

où $R^1$, $R^2$, $R^3$, $R^4$, $R^5$, $R^6$, $R^7$, $R^8$, X, Y, Z, m, n et o ont les significations données dans la revendication 1.

**3.** Procédé selon la revendication 1 pour préparer des composés de formule (3)

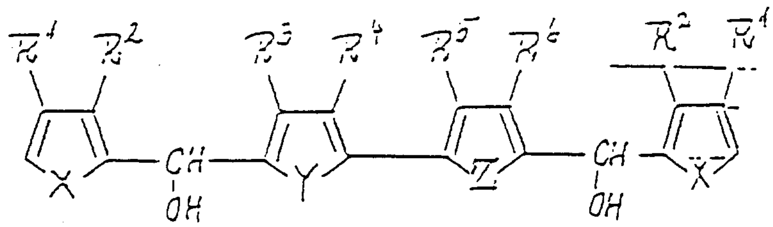

(3) ,

où X, Y, Z, $R^1$, $R^2$, $R^3$, $R^4$, $R^5$ et $R^6$ ont les significations données dans la revendication 1.

**4.** Procédé pour préparer des polymères ayant des unités (hétéro-)arylèneméthines par polycondensation des composés préparés selon la revendication 1, 2 ou 3 en présence d'un agent de condensation choisi dans le groupe des chlorures d'acides, des anhydrides d'acides, des acides forts, des bases et des composés hygroscopiques.

**5.** Procédé selon la revendication 4, où l'on ajoute un agent de dopage avant, pendant ou après la polycondensation, cet agent étant choisi dans le groupe des métaux alcalins, des acides protoniques et des acides de Lewis.

**6.** Procédé selon la revendication 4 par polycondensation de composés de formule (1) pour obtenir des polymères qui comportent des unités de formule (7) :

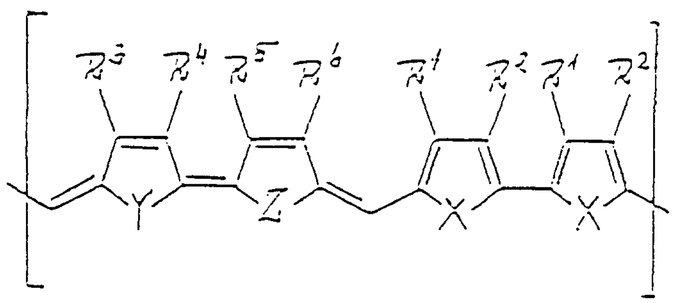

(7) ,

où $R^1$, $R^2$, $R^3$, $R^4$, $R^5$, $R^6$, X, Y, Z ont les significations données dans la revendication 1, à la condition qu'au moins un des cycles relié conformément à la formule (7) par un groupe de formule =CH- se distingue des autres par au moins un des radicaux $R^1$, $R^2$, $R^3$, $R^4$, $R^5$, $R^6$, X, Y, Z.

**7.** Procédé selon la revendication 6, où l'on ajoute un agent de dopage avant, pendant ou après la polycondensation, cet agent étant choisi dans le groupe des métaux alcalins, des acides protoniques et des acides de Lewis.

Fig. 1

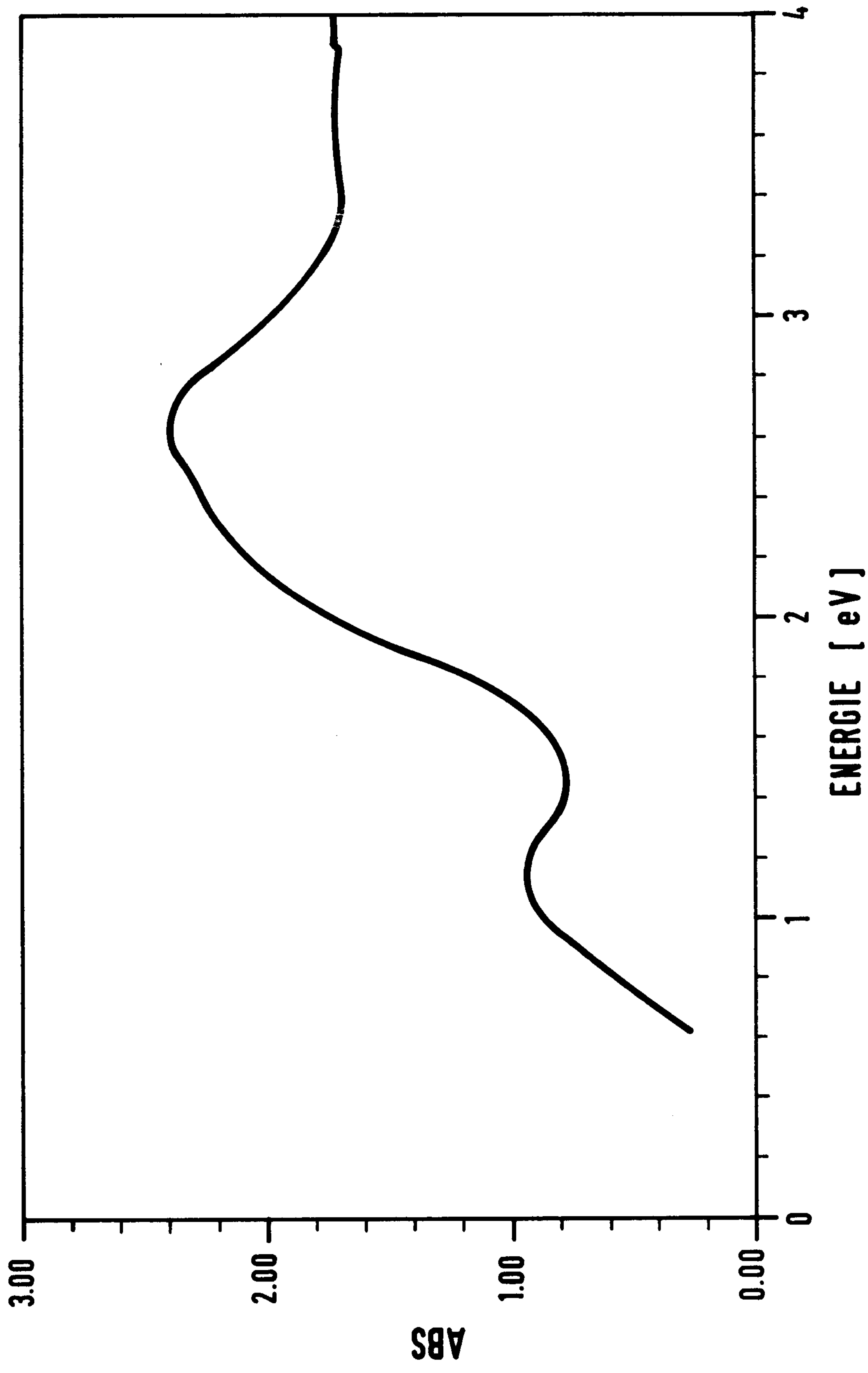

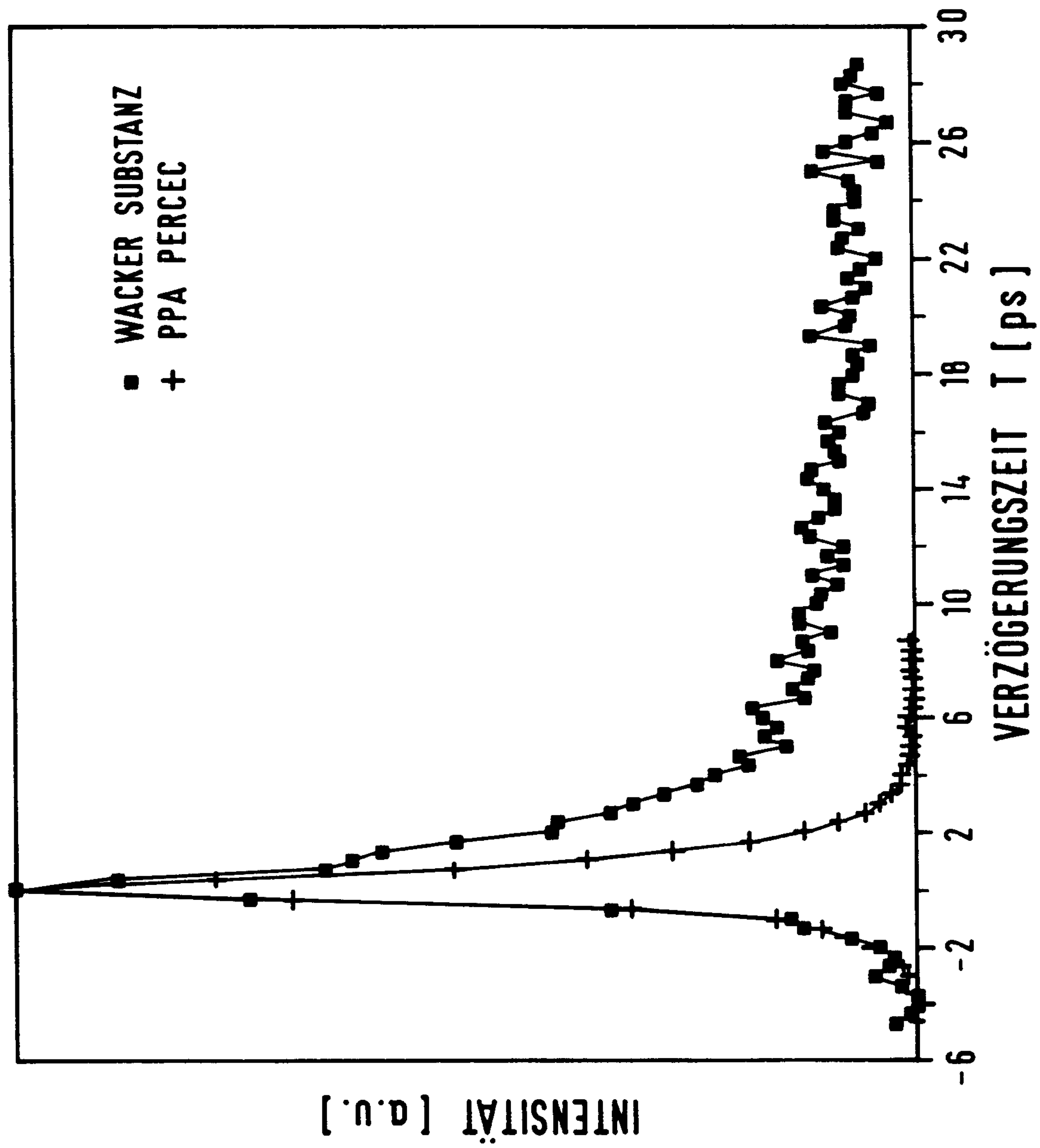
WACKER SUBSTANZ
PPA PERCEC
VERZÖGERUNGSZEIT T [ps]
INTENSITÄT [a.u.]
-6
-2
2
6
10
14
18
22
26
30

Fig. 2